# EUROPEAN PATENT APPLICATION

(11) **EP 4 450 115 A2**
(43) Date of publication of application: **23.10.2024**
(21) Application number: 24170297.6
(22) Date of filing: 15.04.2024
(51) Int. Cl.: A61M 39/06, A61M 39/10, A61M 39/24, A61M 39/20, A61M 1/14

(54) **VASCULAR ACCESS ADAPTOR OF AN EXTRACORPOREAL LIFE SUPPORT SYSTEM**

(30) Priority: 18.04.2023 US 202363460154 P; 28.03.2024 US 202463571016 P; 09.04.2024 US 202418630169
(71) Applicant: CardiacAssist, Inc., Pittsburgh, PA 15238 (US)
(72) Inventor: JAKOB, Rebecca E., Chicago, IL 60655 (US); DACEY, Christopher, Barrington, RI 02806 (US); MELANDER, Mitchell Paul, Pittsburgh, PA 15219 (US); BUSCH, David, 79100 Freiburg (DE)
(74) Representative: Buzzi, Notaro & Antonielli d'Oulx S.p.A.

(57) **Abstract**

An adaptor for use in a blood conduit of an extracorporeal life support system. The adaptor includes a body defining a main passageway and an auxiliary port extending from the body with a passageway of the auxiliary port converging with the main passageway. The auxiliary port includes a hemostasis valve configured to selectively seal around a medical device inserted through the auxiliary port into the main passageway. An elastomeric valve may be positioned in the passageway of the auxiliary port spaced away from the hemostasis valve such that elastomeric valve is located between the hemostasis valve and the main passageway. In some instances, the hemostasis valve may be removably couplable to the auxiliary port.

## Description

### CROSS REFERENCE TO RELATED APPLICATIONS

This application claims the benefit of U.S. Patent Application Serial No. 18/630,169, filed on April 9, 2024, U.S. Provisional Patent Application Serial No. 63/571,016, filed on March 28, 2024, and U.S. Provisional Patent Application Serial No. 63/460,154, filed on April 18, 2023, the disclosures of which are incorporated herein by reference.

### TECHNICAL FIELD

The present disclosure pertains to an adaptor for use in a blood conduit of an extracorporeal life support (ECLS) system or other extracorporeal system. More particularly, the present disclosure pertains to a Y-adaptor including a side port having a hemostasis valve for introducing a medical device into the blood pathway of the ECLS system or other extracorporeal system.

### BACKGROUND

Some medical procedures (e.g., medical procedures which treat cardiac or respiratory disease) may require the use of a life support system that supports cardiac and pulmonary functions by artificially supporting the heart and the lung function. In some instances, this may be carried out by an extracorporeal membrane oxygenation (ECMO) system, also known as an extracorporeal life support (ECLS) system. ECLS is an extracorporeal system which provides both cardiac and respiratory support to a patient whose heart and lungs are unable to provide an adequate amount of gas exchange to sustain life. ECLS works by removing blood from a patient's body to purify and oxygenate the red blood cells while also removing carbon dioxide. The purified and oxygenated blood is then returned to the patient.

ECLS systems may include multiple devices that together form a blood recirculation loop between the patient and a blood oxygenator. For example, some ECLS systems may include a blood reservoir, a blood pump to power blood flow, an oxygenator to oxygenate the blood, a device to filter the blood (which may be included within the oxygenator in some systems), a heat exchanger (to heat and/or cool blood), one or more oxygen sensors positioned at various locations along blood pathways and a control console. It can be appreciated that a blood pathway (e.g., cannula, tubing) may extend from the patient to the blood reservoir, then towards a blood pump, then pass through the oxygenator and close the loop by returning to the patient. Accordingly, the blood pump may assist the heart by pumping blood through the circulation loop, while the oxygenator may assist the lungs by oxygenating blood that is eventually returned to the patient.

During use of the ECLS, it may be desirable to introduce another medical device into the blood pathway to advance the medical device into the patient's cardiovascular system to provide patient monitoring. Accordingly, an adaptor as described herein, could be incorporated with one or more components of the ECLS system and/or connected along the associated tubing of the ECLS to provide access to the blood pathway. Adaptors as described herein, could also be incorporated with one or more components of another extracorporeal system and/or connected along associated tubing of an extracorporeal system.

### BRIEF SUMMARY

This disclosure provides design, material, manufacturing method, and use alternatives for medical devices, including adaptors for a blood pathway of an ECLS system.

A first example is an adaptor for use in a blood conduit of an extracorporeal life support system. The adaptor includes a body defining a main passageway extending therethrough along a longitudinal axis of the body from a first opening at a first end of the body to a second opening at a second end of the body. A side port extends from the body with a passageway of the side port converging with the main passageway. The passageway of the side port has a longitudinal axis extending at an acute angle to the longitudinal axis of the body. The side port includes a hemostasis valve configured to selectively seal around a medical device inserted through the side port into the main passageway. An elastomeric valve is positioned in the passageway of the side port spaced away from the hemostasis valve. The elastomeric valve is located between the hemostasis valve and the main passageway.

Alternatively or additionally to any of the examples above, in another example, the elastomeric valve is a dome valve.

Alternatively or additionally to any of the examples above, in another example, the elastomeric valve is a duckbill valve.

Alternatively or additionally to any of the examples above, in another example, the adaptor includes a vent port extending from the side port. The vent port includes a vent passageway converging with the passageway of the side port.

Alternatively or additionally to any of the examples above, in another example, the vent port is positioned between the hemostasis valve and the main passageway.

Alternatively or additionally to any of the examples above, in another example, the hemostasis valve includes a compressible seal having a lumen extending therethrough, and a compression cap configured to exert a compressive force on the compressible seal to reduce a diameter of the lumen through the compressible seal.

Alternatively or additionally to any of the examples above, in another example, the compression cap includes a stem threadingly engaged to a knob.

Alternatively or additionally to any of the examples above, in another example, the hemostasis valve includes a valve body and the compression cap includes a lip surrounding the valve body.

Alternatively or additionally to any of the examples above, in another example, a volume of the passageway of the side port between the elastomeric valve and the main passageway is 0.02 in³ or less.

Alternatively or additionally to any of the examples above, in another example, the volume of the passageway of the side port between the elastomeric valve and the main passageway is 0.01 in³ or less.

Another example is an adaptor for use in a blood conduit of an extracorporeal life support system. The adaptor includes a body defining a main passageway extending therethrough along a longitudinal axis of the body from a first opening at a first end of the body to a second opening at a second end of the body. A side port extends from the body with a passageway of the side port converging with the main passageway and having a longitudinal axis extending at an acute angle to the longitudinal axis of the body. The side port includes a hemostasis valve configured to selectively seal around a medical device inserted through the side port into the main passageway. An elastomeric valve is positioned in the passageway of the side port between the hemostasis valve and the main passageway. A vent port extends from the side port. The vent port includes a vent passageway converging with the passageway of the side port at a location between the hemostasis valve and the elastomeric valve.

Alternatively or additionally to any of the examples above, in another example, the elastomeric valve is a dome valve.

Alternatively or additionally to any of the examples above, in another example, the elastomeric valve is a duckbill valve.

Alternatively or additionally to any of the examples above, in another example, the hemostasis valve includes a compressible seal having a lumen extending therethrough, and a compression cap configured to exert a compressive force on the compressible seal to reduce a diameter of the lumen through the compressible seal.

Alternatively or additionally to any of the examples above, in another example, the compression cap includes a stem threadingly engaged to a knob.

Alternatively or additionally to any of the examples above, in another example, the hemostasis valve includes a valve body and the compression cap includes a lip surrounding the valve body.

Alternatively or additionally to any of the examples above, in another example, a volume of the passageway of the side port between the elastomeric valve and the main passageway is 0.02 in³ or less.

Alternatively or additionally to any of the examples above, in another example, the volume of the passageway of the side port between the elastomeric valve and the main passageway is 0.01 in³ or less.

Another example is an adaptor for use in a blood conduit of an extracorporeal life support system. The adaptor includes a body defining a main passageway extending therethrough along a longitudinal axis of the body from a first opening at a first end of the body to a second opening at a second end of the body. A side port extends from the body with a passageway of the side port converging with the main passageway and having a longitudinal axis extending at an acute angle to the longitudinal axis of the body. An elastomeric valve is positioned in the passageway of the side port. A vent port extends from the side port. The vent port includes a vent passageway converging with the passageway of the side port. The elastomeric valve is located between the vent port and the main passageway.

Alternatively or additionally to any of the examples above, in another example, the elastomeric valve is a dome valve or a duckbill valve.

Alternatively or additionally to any of the examples above, in another example, the side port includes a hemostasis valve configured to selectively seal around a medical device inserted through the side port into the main passageway.

Alternatively or additionally to any of the examples above, in another example, the hemostasis valve includes a compressible seal having a lumen extending therethrough, and a compression cap configured to exert a compressive force on the compressible seal to reduce a diameter of the lumen through the compressible seal.

Alternatively or additionally to any of the examples above, in another example, the vent passageway of the vent port converges with the passageway of the side port at a location between the hemostasis valve and the elastomeric valve.

Alternatively or additionally to any of the examples above, in another example, a volume of the passageway of the side port between the elastomeric valve and the main passageway is 0.02 in³ or less.

Alternatively or additionally to any of the examples above, in another example, the volume of the passageway of the side port between the elastomeric valve and the main passageway is 0.01 in³ or less.

Another example is a method of accessing a blood pathway of an extracorporeal life support system. The method includes inserting a distal end of an elongate shaft of a medical device through lumen of a compressible seal of a hemostasis valve provided with a side port of an adaptor. The adaptor includes a body defining a main passageway extending therethrough along a longitudinal axis. The main passageway defines a portion of the blood pathway. The side port extends at an acute angle to the longitudinal axis. Thereafter, the method includes advancing the distal end of the elongate shaft through an elastomeric valve positioned in the side port between the hemostasis valve and the main passageway. Thereafter, the method includes advancing the distal end of the elongate shaft into the main passageway of the adaptor to introduce the distal end of the elongate shaft into the blood pathway.

Alternatively or additionally to any of the examples above, in another example, the method includes rotating a compression cap of the hemostasis valve to exert a compressive force on the compressible seal to reduce the diameter of the lumen to seal the compressible seal around the elongate shaft.

Alternatively or additionally to any of the examples above, in another example, the method includes advancing a tubular sheath over the elongate shaft of the medical device such that the tubular sheath extends through the compressible seal of the hemostasis valve and extends through the elastomeric valve with the elongate shaft of the medical device positioned within a lumen of the tubular sheath. Thereafter, the method includes withdrawing the elongate shaft from the side port of the adaptor without the elongate shaft directly contacting the elastomeric valve.

Another example is an adaptor for use in a blood conduit of an extracorporeal life support system. The adaptor includes a body and a side port extending from the body. The body defines a main passageway extending therethrough along a longitudinal axis of the body from a first opening at a first end of the body to a second opening at a second end of the body. The side port defines a passageway converging with the main passageway and having a longitudinal axis extending at an acute angle to the longitudinal axis of the body. At least one aperture extends through a thickness of a side wall of the side port. The side port includes a hemostasis valve configured to selectively seal around a medical device inserted through the side port into the main passageway. An elastomeric valve is positioned in the passageway of the side port spaced away from the hemostasis valve. The elastomeric valve is located between the hemostasis valve and the main passageway.

Alternatively or additionally to any of the examples above, in another example, a surface of the elastomeric valve facing the main passageway is flush with the main passageway of the body.

Alternatively or additionally to any of the examples above, in another example, the surface of the elastomeric valve is a concave surface having a same radius of curvature as an interior surface of the body defining the main passageway.

Alternatively or additionally to any of the examples above, in another example, the at least one aperture comprises a first aperture and a second aperture.

Alternatively or additionally to any of the examples above, in another example, the first aperture and the second aperture are positioned on opposite sides of the body.

Alternatively or additionally to any of the examples above, in another example, the adapter further includes at least one reinforcement member extending between the body and the side port.

Alternatively or additionally to any of the examples above, in another example, the at least one aperture extends through the at least one reinforcement member.

Alternatively or additionally to any of the examples above, in another example, the at least one reinforcement member comprises a first reinforcement member and a second reinforcement member positioned on opposite sides of the body.

Alternatively or additionally to any of the examples above, in another example, the first reinforcement member and the second reinforcement member formed monolithically with the body and the side port.

Alternatively or additionally to any of the examples above, in another example, the adapter further includes at least one slit extending through the elastomeric valve.

Alternatively or additionally to any of the examples above, in another example, there is at least one opening extending through the elastomeric valve.

Another example is a method for forming an elastomeric seal in an adaptor for use in a blood conduit of an extracorporeal life support system. The method includes inserting a first mandrel through a main passageway of a body of an adaptor and inserting a second mandrel into a side passageway of a side port of the adaptor. The side passageway converges with the main passageway. The method further includes injecting a material into the side passageway through one or more apertures extending through a side wall of the side port and curing the material within the side passageway to form an elastomeric seal.

Alternatively or additionally to any of the examples above, in another example, the first mandrel is cylindrical.

Alternatively or additionally to any of the examples above, in another example, an outer diameter of the first mandrel is substantially the same as a diameter of the main passageway.

Alternatively or additionally to any of the examples above, in another example, the second mandrel includes a first cylindrical portion and a second reduced diameter portion.

Alternatively or additionally to any of the examples above, in another example, an outer diameter of the first cylindrical portion is substantially the same as a diameter of the side passageway.

Alternatively or additionally to any of the examples above, in another example, a diameter of the second reduced diameter portion is less than a diameter of the side passageway.

Alternatively or additionally to any of the examples above, in another example, a first end surface of the second reduced diameter portion is concave or planar.

Alternatively or additionally to any of the examples above, in another example, a surface of the elastomeric seal facing the main passageway is concave.

Alternatively or additionally to any of the examples above, in another example, the surface of the elastomeric valve facing the main passageway is flush with an interior wall of the body defining the main passageway.

Another example is an adaptor kit for use in a blood conduit of an extracorporeal life support system. The adaptor kit includes an adaptor including a body defining a non-linear main passageway extending therethrough from a first opening at a first end of the body to a second opening at a second end of the body. The body includes a distal end region defining a distal end region of the main passageway and a proximal end region defining a proximal end region of the main passageway. A longitudinal axis of the distal end region of the main passageway converges with a longitudinal axis of the proximal end region of the main passageway. An auxiliary port extends from the body. A passageway of the auxiliary port converges with the main passageway and has a longitudinal axis extending at an acute angle to the longitudinal axis of the proximal end region of the main passageway. Aan elastomeric valve is positioned in the passageway of the auxiliary port adjacent to the main passageway. The kit also includes a cap and plug assembly configured to be releasably coupled with the auxiliary port of the adaptor, and a hemostasis valve configured to be releasably coupled to the auxiliary port of the adaptor.

Alternatively or additionally to any of the examples above, in another example, the cap and plug assembly includes a plug having a first portion configured to be received within the passageway of the auxiliary port and a second portion configured to at least partially surround a vent port of the adaptor, and a cap configured to be releasably coupled to the second portion of the plug.

Alternatively or additionally to any of the examples above, in another example, the plug includes a lateral cut-out configured to allow the vent port to laterally pass therethrough.

Alternatively or additionally to any of the examples above, in another example, the hemostasis valve includes a housing and a compression cap assembly.

Alternatively or additionally to any of the examples above, in another example, the housing of the hemostasis valve includes one or more protuberances extending from an exterior of the housing.

Alternatively or additionally to any of the examples above, in another example, the one or more protuberances are configured to be received within a mating slot on the auxiliary port.

Alternatively or additionally to any of the examples above, in another example, the longitudinal axis of the passageway of the auxiliary port is parallel with the longitudinal axis of the distal end region of the main passageway.

Alternatively or additionally to any of the examples above, in another example, the longitudinal axis of the passageway of the auxiliary port is co-axial with the longitudinal axis of the distal end region of the main passageway.

Alternatively or additionally to any of the examples above, in another example, a volume of the passageway of the side port between the elastomeric valve and the main passageway is 0.02 in³ or less.

Alternatively or additionally to any of the examples above, in another example, a surface of the elastomeric valve facing the main passageway is flush with the main passageway of the body.

Alternatively or additionally to any of the examples above, in another example, the surface of the elastomeric valve is a concave surface having a same radius of curvature as an interior surface of the body defining the main passageway.

Alternatively or additionally to any of the examples above, in another example, the cap and plug assembly is configured to be decoupled from the auxiliary port of the adaptor prior to coupling the hemostasis valve to the auxiliary port of the adaptor.

Another example is an adaptor for use in a blood conduit of an extracorporeal life support system. The adaptor includes a body defining a main passageway extending therethrough from a first opening at a first end of the body to a second opening at a second end of the body. A side port extends from the body. A passageway of the side port converges with the main passageway and has a longitudinal axis extending at an acute angle to a longitudinal axis of the body. The side port includes a hemostasis valve configured to selectively seal around a medical device inserted through the side port into the main passageway. An elastomeric valve is positioned in the passageway of the side port spaced away from the hemostasis valve. The elastomeric valve is located between the hemostasis valve and the main passageway.

Alternatively or additionally to any of the examples above, in another example, a volume of the passageway of the side port between the elastomeric valve and the main passageway is 0.02 in³ or less.

Alternatively or additionally to any of the examples above, in another example, the adaptor includes a vent port extending from the side port, wherein the vent port includes a vent passageway converging with the passageway of the side port, wherein the vent port is positioned between the hemostasis valve and the main passageway.

Alternatively or additionally to any of the examples above, in another example, the hemostasis valve includes a compressible seal having a lumen extending therethrough, and a compression cap configured to exert a compressive force on the compressible seal to reduce a diameter of the lumen through the compressible seal.

Alternatively or additionally to any of the examples above, in another example, the compression cap includes a stem threadingly engaged to a knob.

The above summary of some embodiments is not intended to describe each disclosed embodiment or every implementation of the present disclosure. The Figures, and Detailed Description, which follow, more particularly exemplify some of these embodiments.

### BRIEF DESCRIPTION OF THE DRAWINGS

The disclosure may be more completely understood in consideration of the following detailed description in connection with the accompanying drawings, in which:
FIG. 1 illustrates an exemplary extracorporeal life support system;
FIG. 2 is a perspective view of an exemplary adaptor for use in an extracorporeal life support system;
FIG. 3 is an exploded view of components of the adaptor of FIG. 2;
FIG. 4 is a cross-sectional view of the adaptor of FIG. 2;
FIG. 5 is a perspective view of an exemplary hemostasis valve of the adaptor of FIG. 2;
FIG. 6 is an exploded view of the hemostasis valve of FIG. 5;
FIG. 7 is a cross-sectional view of the hemostasis valve of FIG. 5;
FIG. 8 is a cross-sectional view of the adaptor of FIG. 2 during use;
FIG. 9 illustrates aspects of withdrawing an elongate shaft of a medical device from the adaptor of FIG. 2;
FIG. 10 is a perspective view of another exemplary adaptor for use in an extracorporeal life support system;
FIG. 11 is a cross-sectional view of the adaptor of FIG. 10 with a seal removed;
FIG. 12 is a cross-sectional view of the adaptor of FIG. 10;
FIG. 13 is a cross-sectional view of the adaptor of FIG. 10 during formation of an elastomeric valve;
FIG. 14A is a perspective view of an illustrative elastomeric valve;
FIG. 14B is a cross-sectional view of the elastomeric valve of FIG. 14A;
FIG. 15A is a perspective view of another illustrative elastomeric valve;
FIG. 15B is a cross-sectional view of the elastomeric valve of FIG. 15A;
FIG. 16 is a partial cross-sectional view of another illustrative elastomeric valve;
FIG. 17 is a partial cross-sectional view of another illustrative elastomeric valve;
FIGS. 18A-18D are schematic view of illustrative openings in the elastomeric valve;
FIG. 19 is a perspective view of another illustrative adaptor and hemostasis valve;
FIG. 20 is a perspective view of the illustrative adaptor of FIG. 19 having a cap and plug assembly in a partially assembled configuration;
FIG. 21 is a cross-sectional view of the illustrative adaptor of FIG. 19 having a cap and plug assembly in a partially assembled configuration;
FIG. 22 is a perspective view of the illustrative adaptor of FIG. 19 having a cap and plug assembly in a second partially assembled configuration;
FIG. 23 is a perspective view of the illustrative adaptor of FIG. 19 having a cap and plug assembly in an unassembled configuration;
FIG. 24 is a side view of the illustrative adaptor of FIG. 19 assembled with a hemostasis valve;
FIG. 25 is a cross-sectional view of the illustrative adaptor and hemostasis valve of FIG. 24;
FIG. 26 is an exploded perspective view of the illustrative adaptor and hemostasis valve of FIG. 24;
FIG. 27 is an exploded perspective view of the illustrative hemostasis valve of FIG. 24; and
FIG. 28 is a cross-sectional view of the illustrative hemostasis valve of FIG. 24.

While the disclosure is amenable to various modifications and alternative forms, specifics thereof have been shown by way of example in the drawings and will be described in detail. It should be understood, however, that the intention is not to limit the disclosure to the particular embodiments described. On the contrary, the intention is to cover all modifications, equivalents, and alternatives falling within the spirit and scope of the disclosure.

### DETAILED DESCRIPTION

For the following defined terms, these definitions shall be applied, unless a different definition is given in the claims or elsewhere in this specification.

All numeric values are herein assumed to be modified by the term "about," whether or not explicitly indicated. The term "about" generally refers to a range of numbers that one of skill in the art would consider equivalent to the recited value (i.e., having the same function or result). In many instances, the terms "about" may include numbers that are rounded to the nearest significant figure.

The recitation of numerical ranges by endpoints includes all numbers within that range (e.g., 1 to 5 includes 1, 1.5, 2, 2.75, 3, 3.80, 4, and 5).

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

It is noted that references in the specification to "an embodiment", "some embodiments", "other embodiments", etc., indicate that the embodiment described may include one or more particular features, structures, and/or characteristics. However, such recitations do not necessarily mean that all embodiments include the particular features, structures, and/or characteristics. Additionally, when particular features, structures, and/or characteristics are described in connection with one embodiment, it should be understood that such features, structures, and/or characteristics may also be used connection with other embodiments whether or not explicitly described unless clearly stated to the contrary.

The following detailed description should be read with reference to the drawings in which similar structures in different drawings are numbered the same. The drawings, which are not necessarily to scale, depict illustrative embodiments and are not intended to limit the scope of the disclosure.

In a normal heart, blood circulates via a closed path whereby deoxygenated (venous) blood enters the right atrium via both the superior vena cava and inferior vena cava. The venous blood then passes through the right ventricle and is pumped via the pulmonary artery to the lungs, where it absorbs oxygen. After absorbing oxygen in the lungs, the blood becomes oxygenated arterial blood. The oxygenated arterial blood is then returned via the pulmonary veins to the left atrium and is passed to the left ventricle. The oxygenated arterial blood is then pumped through the aorta and eventually throughout the body.

It can be appreciated that if the lungs are incapable of sufficiently oxygenating blood, an oxygenator located outside the body may be used to oxygenate the blood. As discussed above, extracorporeal life support (ECLS) is a breathing and heart pumping life support system that may be utilized to support patients while medical treatments are performed to treat their underlying illness. When supported via an ECLS system, oxygenation of the patient's blood and removal of carbon dioxide may occur outside the body.

ECLS is generally performed using a heart-lung bypass system, which may be referred to as a "circuit." The circuit may include one or more tubing pathways designed to transfer blood from a patient's body to the oxygenator and back into the patient. As described above, the oxygenator may add oxygen to the blood while also removing carbon dioxide (e.g., the oxygenator performs the function of a healthy lung).

In some examples, an ECLS circuit may include a blood pump, oxygenator, tubing pathways (for transfer to and from the body), flow and/or pressure sensors, a heat exchanger (to cool and/or heat the blood), a computing console, and arterial and/or venous access points for the collection of blood in the circuit. It can be appreciated that the function of the blood pump is to generate blood flow within the ECLS circuit (e.g., circulate blood from the patient to the oxygenator and back to the patient). The blood pump may be positioned in the tubing pathway between the patient and the oxygenator, or at a different location of the blood pathway. In some ECLS systems a roller pump may be utilized to generate blood flow within the ECLS circuit. However, in other ECLS systems, other blood pumps, including centrifugal pumps may be utilized to generate blood flow within the ECLS circuit.

In some ECLS systems, the oxygenator may include a housing having multiple chambers or pathways separated by a semi-permeable membrane, whereby the patient's blood may flow through one chamber or pathway, while an oxygen gas mixture (i.e., sweep gas) flows through another chamber or pathway. The semi-permeable membrane may include multiple microporous hollow fibers, each fiber having a lumen extending therethrough through which the oxygen gas mixture flows. The gas exchange may occur via diffusion of the gases across multiple microporous fibers, whereby oxygen moves from the inside of the hollow fibers into the blood while carbon dioxide diffuses from the blood into the interior of the hollow fibers, where it is swept away by the sweep gas flowing through the fiber. This gas exchange allows for oxygenation of venous blood and removal of carbon dioxide. In some ECLS systems, the oxygenator may include an integrated heat exchanger, or a stand-alone heat exchanger, that allows circulating blood to be cooled and/or warmed prior to returning to the patient.

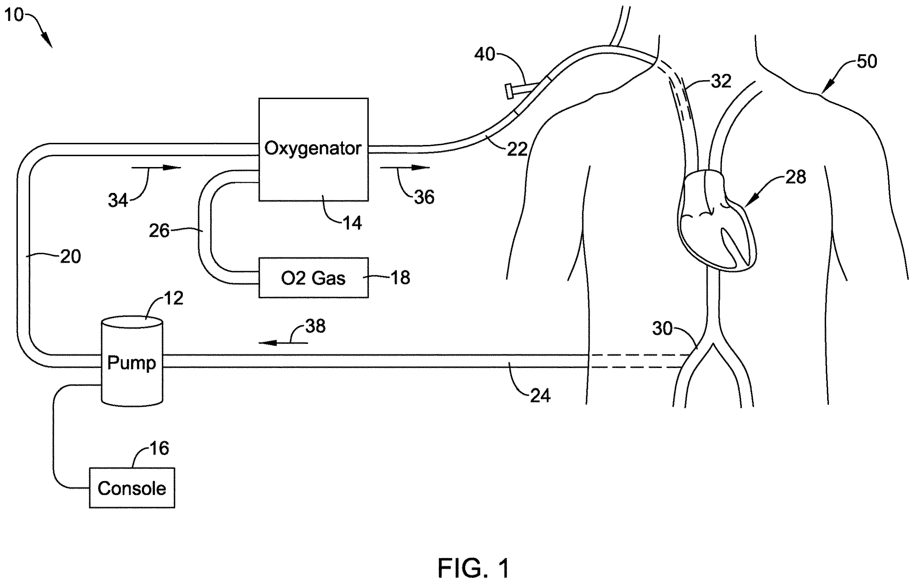

FIG. 1 illustrates an ECLS system 10 including a blood pump 12 designed to draw deoxygenated blood from a patient 50 and propel the blood to an oxygenator 14. Further, after the blood passes through the semi-permeable membrane of the oxygenator 14, the post-oxygenated blood may return to the patient 50.

FIG. 1 illustrates a type of ECLS life support system designed to support lung function. This type of ECLS system may be referred to as a veno-venous (VV) ECLS life support system. The veno-venous ECLS system shown in FIG. 1 may include two separate cannulation sites (e.g., sites at which tubular members are inserted into the patient's body). For example, FIG. 1 shows a first cannulation site in which a cannula is inserted into the femoral vein 30. While not illustrated in FIG. 1, the cannula inserted into the femoral vein 30 may be tracked into the inferior vena cava, for example, whereby it may be utilized to drain deoxygenated blood from the patient. As illustrated in FIG. 1, a cannula inserted into the femoral vein 30 may be connected to a tubular pathway 24 (e.g., a length of tubing) which may be coupled to the pump 12. Therefore, it can be appreciated that the pump 12 may be utilized to remove (e.g., pull, drain, draw, etc.) deoxygenated blood from the patient 50.

FIG. 1 further illustrates that the ECLS life support system may further include a tubular pathway 20 (e.g., a length of tubing) extending between the pump 12 and the oxygenator 14. The tubular pathway 20 may be an extension of the tubular pathway 24. For example, the tubular pathway 20 and the tubular pathway 24 may be a continuous tubular member which passes through and engages with the pump 12. Alternatively, the tubular pathway 20 and the tubular pathway 24 may be separate tubular members, each of which include an end region that connects to the pump 12. In other instances, the pump 12 may be directly attached to or integral with the oxygenator 14, thus eliminating the need for the tubular pathway 20 therebetween. The tubular pathways 20/24 may be referred to as a drainage line removing deoxygenated blood from the patient.

Additionally, the veno-venous ECMO system shown in FIG. 1 shows a second cannulation site in which a cannula is inserted into the right jugular vein 32 to return oxygenated blood to the heart 28 of the patient 50. While not illustrated in FIG. 1, the cannula inserted into the right jugular vein 32 may be tracked into the right atrium of the heart 28, for example, whereby it may be utilized to return oxygenated blood to the patient 50. As illustrated in FIG. 1, a cannula inserted into the right jugular vein 32 may be connected to a tubular pathway 22 (e.g., a length of tubing) which may be coupled to the oxygenator 14. It can be appreciated that the pump 12 may be utilized to propel oxygenated blood from the oxygenator 14 to the patient 50. The tubular pathway 22 may be referred to as a return line returning oxygenated blood back to the patient 50.

FIG. 1 further illustrates that the ECLS system 10 may further include an oxygen source 18 coupled to the oxygenator 14. The oxygen source 18, in some instances, may include an oxygen tank which is coupled to the oxygenator 14 via the tubular pathway 26. The oxygenator 14 may draw oxygen from the oxygen source 18 to oxygenate blood passing through the semi-permeable membrane of the oxygenator 14.

As discussed herein, the ECLS system 10 may include a blood pump 12 designed to draw deoxygenated blood from the inferior vena cava of a patient 50 (the direction of deoxygenated blood out of the patient is shown by arrow 38) and propel the blood to an oxygenator 14 (the direction of deoxygenated blood from the pump to the oxygenator is shown by arrow 34). After the deoxygenated blood enters the oxygenator 14 it may pass through the semi-permeable membrane of the oxygenator 14 whereby red blood cells absorb oxygen (drawn from the oxygen source 18) and carbon dioxide is released. After blood passes through the semi-permeable membrane of the oxygenator 14, the post-oxygenated blood may return to the patient 50 (the direction of post-oxygenated blood going to the patient is shown by arrow 36) through the tubular pathway 22. After passing through a cannula inserted into the right jugular vein 32, the post-oxygenated blood may be released in the right atrium of the heart 28 of the patient 50.

Additionally, FIG. 1 illustrates that in some examples, the ECLS system 10 may include a controller or console 16 coupled to various components of the ECLS system 10. For example, FIG. 1 illustrates that the console 16 may be coupled to the pump 12. However, it is also contemplated that the console 16 may be coupled to the oxygenator 14 and/or the oxygen source 18. Further, the console 16 may include a computing device. The console 16 may further include, among other suitable components, a processor, display, memory, and an I/O unit.

Furthermore, the ECLS system 10 may include an adaptor 40 allowing access to the blood pathway for introducing a medical device into the blood pathway. As shown in FIG. 1, the adaptor 40 may be provided along the tubular pathway 22 returning oxygenated blood back to the patient 50. It is noted, however, in other embodiments the adaptor may be positioned at a different location along the blood pathway, if desired. In some instances, the adaptor 40 may be located at or be included as a hub and/or connector of a cannula defining a portion of the tubular pathway 22, or the adaptor 40 may be positioned between two lengths of tubing defining a portion of the tubular pathway 22. The adaptor 40 will be further described herein.

While the above discussion describes a veno-venous ECLS system, it is contemplated that an adaptor, as described herein, may be utilized with different ECLS systems. For example, an adaptor, as described herein, may be utilized with a veno-atrial ECLS system. Furthermore, an adaptor, as described herein, may be incorporated with an access cannula, such as a dual lumen access cannula, used in conjunction with an ECLS system, which may have a variety of cannulation configurations. In some examples, the adaptor, as described herein, may be used with any extracorporeal system or procedure and is not limited to ECLS systems.

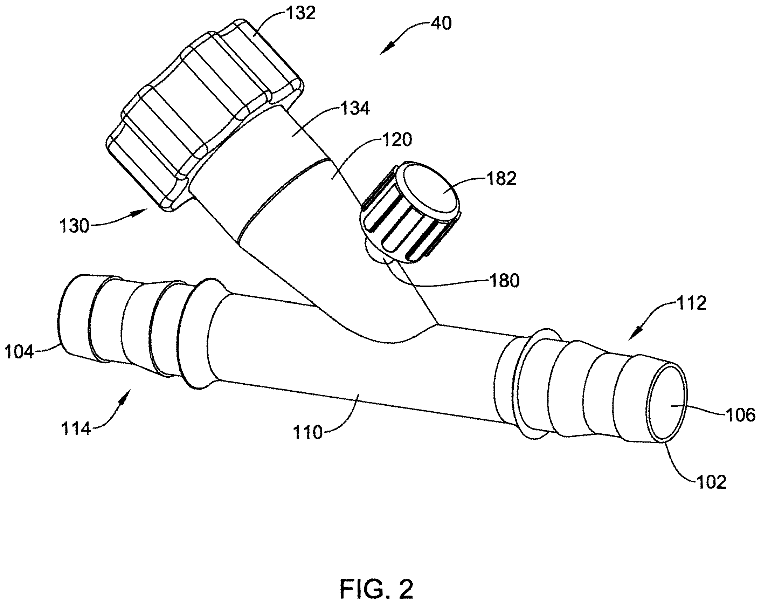

FIGS. 2-4 illustrate aspects of the adaptor 40. As shown in FIG. 2, the adaptor 40 may include a body 110 having a first end 102 and a second end 104. The body 110 may define a main passageway 106 extending through the body 110 along a longitudinal axis X. The main passageway 106 may open out at a first opening at the first end 102 and a second opening at the second end 104. Thus, the main passageway 106 of the adaptor 40 may extend from the first opening at the first end 102 of the body 110 to the second opening at the second end 104 of the body 110. In some instances, the first end 102 of the body 110 may include a connector 112 and/or the second end 104 of the body may include a connector 114 configured to connect the adaptor 40 to a conduit (e.g., tubing or cannula) of the ECLS system. However, in other instances the body 110 may be incorporated as a component of a cannula, such as an access cannula. The connectors 112, 114 are shown as barb fittings configured to be inserted into the lumen of a tubular member (e.g., tubing). The barb fittings may include one or more projecting annular ridges configured to form an interface fit against an inner surface of a tubular member (e.g., tubing). The barb fittings may be any desired size to fit into any desired size of medical tubing. For instance, the barb fittings may be 3/8-inch diameter barb fittings configured to be inserted into a 3/8-inch diameter medical tubing. In other instances, the barb fittings may be of a different size, such as 1/4-inch diameter, 5/16-inch diameter, 1/2-inch diameter, or other desired size to accommodate a corresponding size of medical tubing. In some instances, the connector 112 may be the same size and/or configuration as the connector 114. However, in other instances the connector 112 may be a different size than the connector 114. For instance, in some instances, the connector 112 may be a 3/8-inch barb fitting and the connector 114 may be a 1/4-inch barb fitting. In other instances, the connectors 112, 114 may be threaded connections configured to threadingly engage a mating threaded structure, such as a threaded structure provided with a tubular member (e.g., a luer connector, etc.).

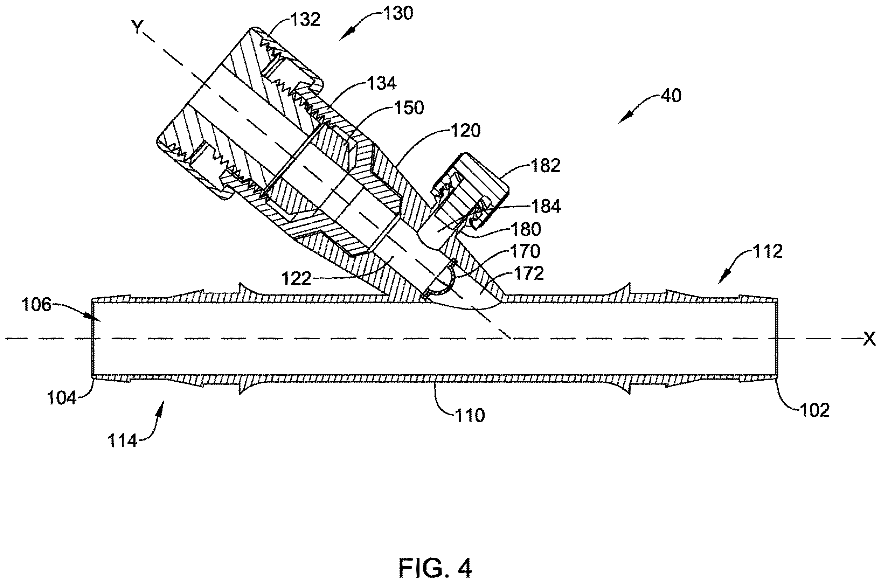

The adaptor 40 may also include a side port 120 (i.e., auxiliary port) extending from the body 110. The side port 120 may include a body defining a passageway 122 extending therein. In some instances, the body of the side port 120 may be a monolithic portion of the body 110, or the body of the side port 120 may be formed separately from the body 110 and fixedly secured thereto. The side port 120 may extend from the body 110 at any desired angle. For example, the side port 120, including the passageway 122 thereof, may extend at an acute angle from the body 110 along a longitudinal axis Y, as shown in FIG. 4. However, in other embodiments the side port 120 may extend at a perpendicular angle from the body 110, if desired. The passageway 122 of the side port 120 may extend along the longitudinal axis Y and converge with the main passageway 106 of the body 110, such as at an acute angle. The passageway 122 of the side port 120 may converge with the main passageway 106 at a location between the first end 102 and the second end 104 of the body 110.

The side port 120 may include a hemostasis valve 130 configured to permit access to the passageway 122 of the side port 120 and into the main passageway 106. Namely, an elongate shaft of a medical device may be passed through the hemostasis valve 130 and into the main passageway 106, which defines a portion of a blood conduit, while preventing appreciable amounts of blood from passing out through the hemostasis valve 130.

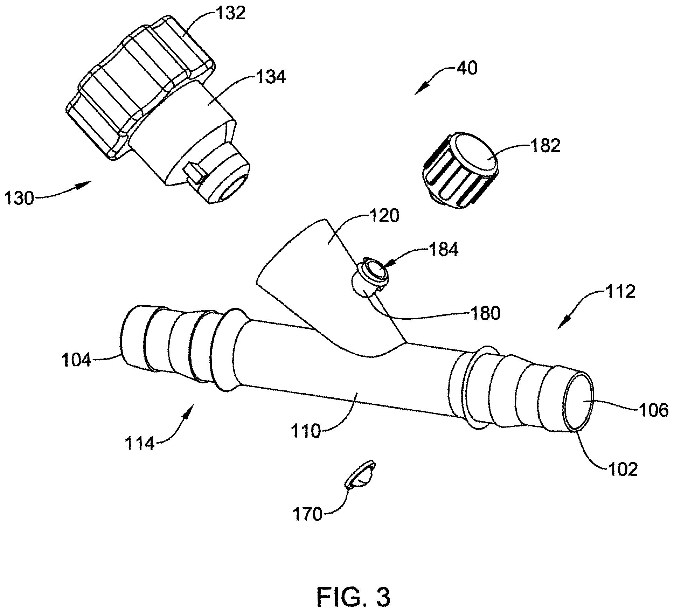

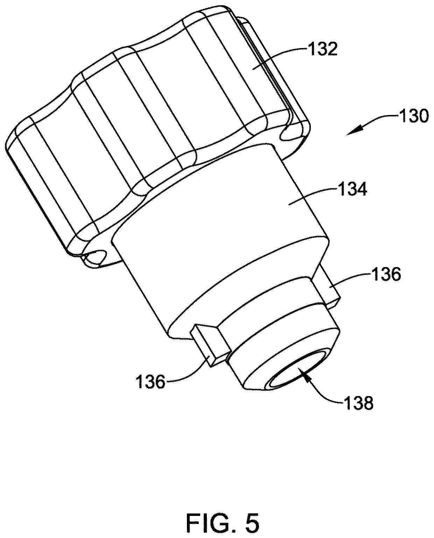

The hemostasis valve 130 may include a housing 134 secured to, incorporated with, extending from, or otherwise positioned along the passageway 122 of the side port 120. For example, the housing 134 of the hemostasis valve 130 may be adhesively bonded to the body of the side port 120. For instance, the housing 134, or at least a portion thereof, may extend into the body of the side port 120 and be adhesively bonded to an inner surface of the body of the side port 120. As shown in FIG. 3, as well as in FIG. 5, the housing 134 may include one or more, or a plurality of protuberances 136 projecting from an exterior surface of the housing 134 which mate in a corresponding channel, groove, notch or recess formed in the body of the side port 120 to facilitate securement and/or rotational orientation between the hemostasis valve 130 and the side port 120.

In other instances, the housing 134 of the hemostasis valve 130 may be integrated into the body 110 of the adaptor 40 as a monolithic portion of the side port 120. In other words, in some instances the side port 120, formed as a monolithic portion of the body 110 of the adaptor 40, may define the housing 134 of the hemostasis valve 130 as an integral portion thereof.

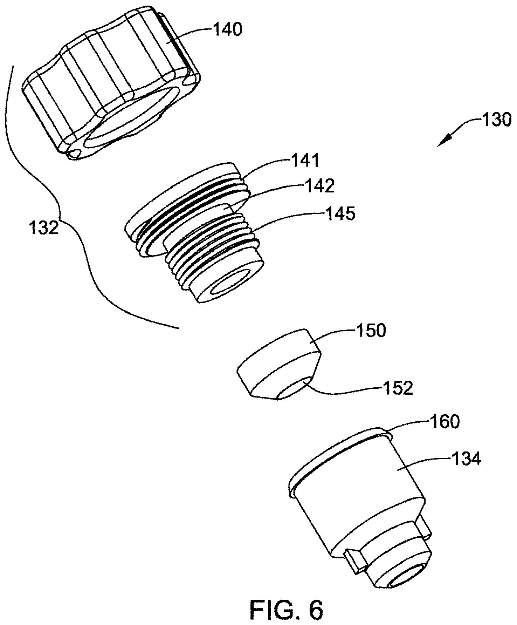

The hemostasis valve 130 may also include a rotatable compression cap 132 that is rotatable relative to the housing 134 and the side port 120. As will be described further herein in association with FIGS. 5-7, the compression cap 132 may be rotated to compress the seal of the hemostasis valve 130 around an elongate shaft of a medical device inserted through the hemostasis valve 130.

The adaptor 40 may also include a vent port 180 having a vent pathway 184 in communication with the passageway 122 of the side port 120. For instance, the vent pathway 184 of the vent port 180 may converge with the passageway 122 of the side port 120 at a location between the hemostasis valve 130 and the main passageway 106. In some instances, the vent pathway 184 of the vent port 180 may converge with the passageway 122 of the side port 120 at a location between the hemostasis valve 130 and an elastomeric valve 170 positioned along the passageway 122 of the side port 120 proximate the main passageway 106.

The vent port 180 may include a vent cap 182, which may be removed to open the vent pathway 184 to the ambient environment to vent fluid (e.g., air) from the passageway 122 of the side port 120. In some instances, the vent cap 182 may include a threaded portion (e.g., female threads) threadingly engaged to a mating threaded portion (e.g., male threads) of the vent port 180. During use, the vent cap 182 may be removed and a syringe may be attached to the threads of the vent port 180. The syringe may then be used to remove any air ingression in the passageway 122.

The elastomeric valve 170 may be positioned along the passageway 122 of the side port 120 between the hemostasis valve 130 and the main passageway 106. In some instances, the vent port 180 may be positioned between the hemostasis valve 130 and the elastomeric valve 170, with the hemostasis valve 130 spaced apart from the elastomeric valve 170 by a portion of the passageway of the side port 120. The elastomeric valve 170 may be a normally closed valve, having a closed configuration in an equilibrium state such that fluid is substantially prevented from passing through the elastomeric valve 170 in its closed configuration. The elastomeric valve 170 may be configured to allow the elongate shaft of a medical device to pass through the elastomeric valve 170 when the elongate shaft of the medical device is introduced through the passageway 122 of the side port 120 and advanced into the main passageway 106. The elastomeric valve 170 may be sufficiently flexible to deform as the elongate shaft of the medical device is passed therethrough and seal around an outer peripheral of the elongate shaft of the medical device to substantially prevent fluid (e.g., blood) from passing across the elastomeric valve 170 from the main passageway 106. The elastomeric valve 170 may be formed of silicone, polyurethane, or other flexible polymeric material. In some instances, the elastomeric valve 170 may be a dome valve, a duckbill valve, a slit valve, or any other desired valve configured to seal around an elongate shaft of a medical device when passed therethrough.

The elastomeric valve 170 may be positioned proximate the main passageway 106 to reduce the volume of the passageway 122 of the side port 120 between the elastomeric valve 170 and the main passageway 106. Reducing or minimizing the volume of the passageway 122 of the side port 120 between the elastomeric valve 170 and the main passageway 106 may effectively reduce and/or minimize the stagnation or pooling of blood in the passageway 122. For instance, the volume of the passageway 122 of the side port 120 between the elastomeric valve 170 and the main passageway 106, called a stagnation volume 172, may be 0.03 in³ or less, 0.02 in³ or less, or 0.01 in³ or less, in some embodiments.

Further aspects of the hemostasis valve 130 are illustrated in FIGS. 5-7. The hemostasis valve 130 may include a lumen 138 extending therethrough for the passage of an elongate shaft of a medical device. The lumen 138 of the hemostasis valve 130 may be axially aligned with the longitudinal axis of the passageway 122 of the side port 120.

The hemostasis valve 130 may include a plurality of components assembled together. For instance, the hemostasis valve 130 may include the compression cap 132 rotatably coupled to the housing 134 of the hemostasis valve 130 to selectively exert a compressive force on a compressible seal 150 positioned in the bore of the housing 134. The compressible seal 150 may include a lumen 152 extending therethrough axially aligned with the lumen 138 of the hemostasis valve 130. The lumen 152 may have a suitable diameter in an open state allowing an elongate shaft of a medical device to pass therethrough. For example, the lumen 152 may have a diameter in an open state of about 15 F (5 millimeters), about 13 F (4.33 millimeters), about 12 F (4 millimeters), about 10 F (3.33 millimeters), or about 9 F (3 millimeters), in some instances. The lumen 152 may be configured to close around an elongate shaft of a medical device via rotation of the compression cap 132 which compresses the compressible seal 150. Thus, the lumen 152 of the compression cap 132 may have a diameter in a closed state which is less than the diameter of the lumen 152 in the open state. In some instances, the compression cap 132 may be configured such that the lumen 152 is able to be completely closed off (e.g., have a diameter of 0 F (0 millimeters) in a completely closed state.

In some instances, the compression cap 132 may be formed of multiple components assembled together. In some instances, forming the compression cap 132 from multiple components may permit the compression cap 132 to be assembled with the housing 134 and the compressible seal 150, but thereafter preclude the compression cap 132 from being removed from the housing 134. For instance, the compression cap 132 may include a knob 140 and a stem 142, formed as separate components, and then coupled together when assembled with the housing 134 of the hemostasis valve 130. In the illustrated embodiment, the knob 140 may include a threaded portion 143 configured to threadingly engage a mating threaded portion 141 of the stem 142. For instance, the knob 140 may include female threading configured to engage with male threading of the stem 142. After being assembled together, the knob 140 may be fixedly secured (e.g., adhesive bonded, etc.) to the stem 142 to prevent separation of the components.

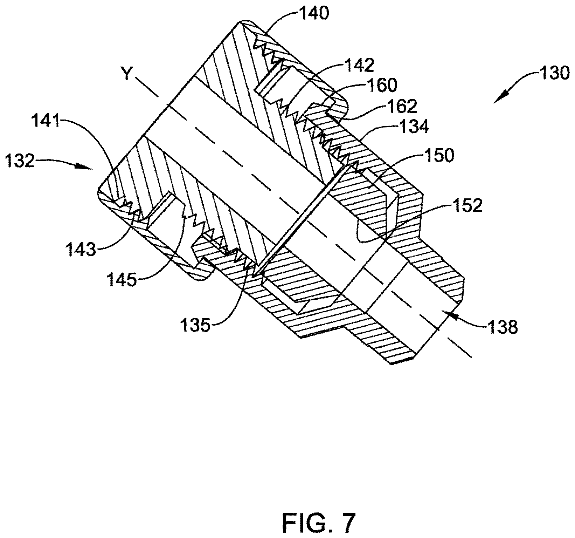

As shown in FIG. 7, the housing 134 of the hemostasis valve 130 may include a rim 160, such as a radially outward projecting rim extending around a first end of the housing 134. In some instances the rim 160 may extend continuously around the first end of the housing 134. In other instances, the rim 160 may extend discontinuously around the first end of the housing 134. The compression cap 132, such as the knob 140 of the compression cap 132, may include a lip 162, such as a radially inward projecting lip 162 extending around the compression cap 132, such as a first end of the knob 140. The lip 162 may be sized such that the rim 160 cannot pass through the opening defined by the lip 162. Such a configuration may prevent the compression cap 132 from being removed from the housing 134 of the hemostasis valve 130.

During assembly, compressible seal 150 may be positioned in the bore of the housing 134, the stem 142 may then be inserted into the bore of the housing 134 above the compressible seal 150 from the second, upper end of the housing 134. The threaded portion 145 (e.g., male threaded portion) of the stem 142 may be threaded into a threaded portion 135 (e.g., female threaded portion) of the bore of the housing 134 to capture the compressible seal 150 in the bore of the housing 134. The knob 140 may also be passed over the housing 134 from the lower, first end of the housing 134. The threaded portion 143 of the knob 140 may then be threadingly engaged with the threaded portion 141 of the stem 142 to couple the knob 140 to the stem 142. As noted above, in some instances, the knob 140 may then be fixedly secured (e.g., adhesive bonded, etc.) to the stem 142 to prevent separation of the components.

During use, the compression cap 132 may be rotated relative to the housing 134, thereby threadably advancing the compression cap 132 in the axial direction to exert a compressive force against the compressible seal 150. For instance, rotation of the compression cap 132 may threadingly advance the threaded portion 145 of the stem 142 along the threaded portion 135 of the bore of the housing 134, with an end surface of the stem 142 of the compression cap 132 pressing against an end surface of the compressible seal 150. The compressive force causes compressible seal 150 to deform, thereby reducing the diameter of the lumen 152 through the compressible seal 150. For instance, the compressive force may cause the length of the compressible seal 150 to be reduced as the diameter of the lumen 152 of the compressible seal 150 is reduced.

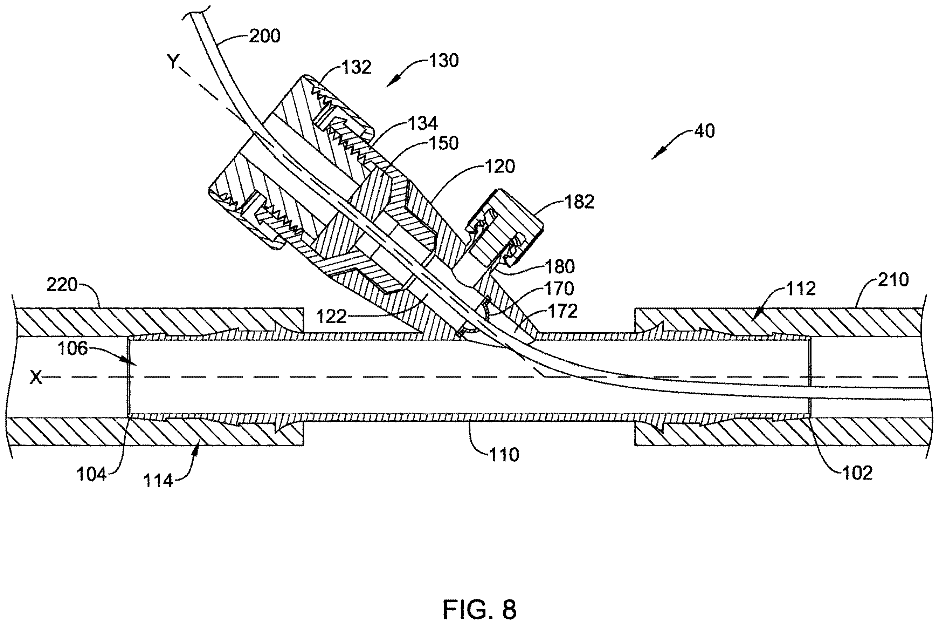

FIG. 8 illustrates the adaptor 40 positioned along a blood conduit, such as positioned between a first length of medical tubing 210 and a second length of medical tubing 220. The first length of medical tubing 210 may be coupled to the first end 102 of the body 110 of the adaptor 40 and the second length of medical tubing 220 may be coupled to the second end 104 of the body 110 of the adaptor 40 with the blood pathway passing therethrough.

In use, an elongate shaft 200 of a medical device is inserted through the lumen of the compressible seal 150 of the hemostasis valve 130 and into the passageway 122 of the side port 120. The elongate shaft 200 of the medical device passes through the passageway 122 and is advanced across the elastomeric valve 170 positioned in the passageway 122 of the side port 120. For example, the elongate shaft 200 may be passed through an opening of the elastomeric valve 170 and cause the elastomeric valve 170 to flex and/or deform to permit the elongate shaft 200 to be advanced therethrough. The elastomeric valve 170 may seal around an outer perimeter of the elongate shaft 200 and substantially prevent blood from entering the passageway 122 of the side port 120 from the main passageway 106 acting as a portion of the blood pathway. Thereafter, the elongate shaft 200 of the medical device passes into the main passageway 106 of the body 110 of the adaptor 40 and into the blood pathway. The elongate shaft 200 may be advanced along the blood pathway into the body of the patient for a diagnostic and/or therapeutic procedure. The compressible seal 150 may be compressed or tightened around the perimeter of the elongate shaft 200 to prevent blood from leaking past the elongate shaft 200. For instance, as discussed above, the compression cap 132 may be rotated to exert a compressive force against the compressible seal 150 to seal the compressible seal 150 around the elongate shaft 200.

The presence of the elastomeric valve 170 proximate the main passageway 106, precludes stagnation of blood along the passageway 122 of the side port 120, even in the absence of any medical device disposed through the hemostasis valve 130. Thus, the passageway 122 of the side port 120 may be substantially free of blood throughout use of the adaptor 40.

In some instances, the vent cap 182 may be removed and a syringe may be attached to the vent port 180. The syringe may be used to remove any air ingression in the passageway 122 such that air does not enter the main passageway 106 into the blood pathway.

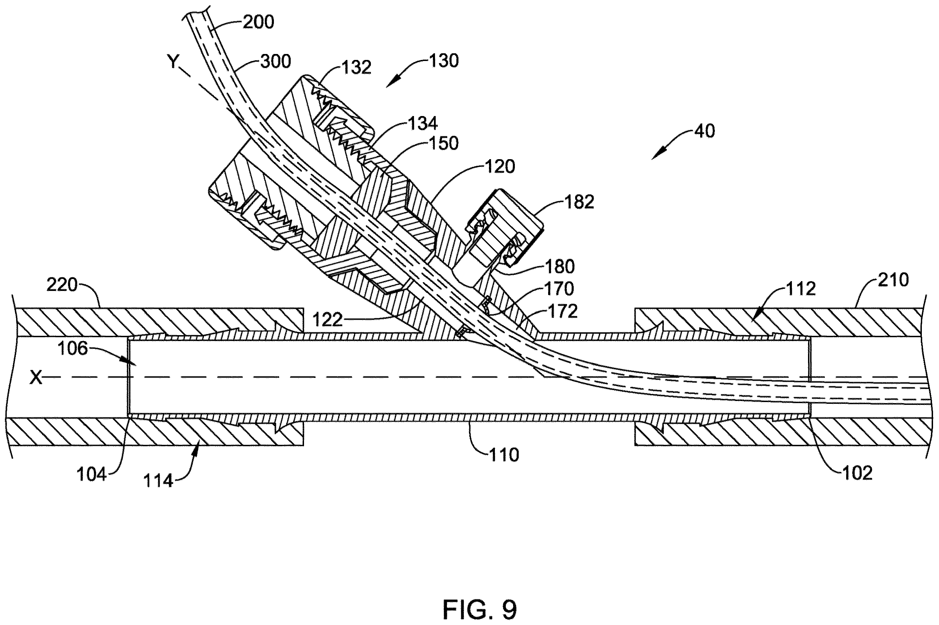

When it is desired to remove the elongate shaft 200 from the blood pathway, the elongate shaft 200 may be withdrawn from the side port 120, such as by being retracted from the elastomeric valve 170 and then retracted from the hemostasis valve 130. In some instances, as shown in FIG. 9, in order to preclude the potential of scraping blood off of the inserted elongate shaft 200 as the elongate shaft 200 is withdrawn from the elastomeric valve 170, a tubular sheath 300 may be advanced distally over the elongate shaft 200 (e.g., surrounding the elongate shaft 200) through the hemostasis valve 130 and the elastomeric valve 170 to provide an interface between the elongate shaft 200 and the portions of the hemostasis valve 130 and/or the elastomeric valve 170 otherwise contacting the outer surface of the elongate shaft 200. In some instances, the tubular sheath 300 may be advanced into the main passageway 106 and/or the blood pathway as the tubular sheath 300 is tracked or advanced distally over the elongate shaft 200. With the tubular sheath 300 extending through the hemostasis valve 130 and/or the elastomeric valve 170, the elongate shaft 200 may be isolated from directly contacting the compressible seal 150 of the hemostasis valve 130 and/or the elastomeric valve 170. Thereafter, the elongate shaft 200 may be withdrawn proximally from the adaptor 40 without the elongate shaft 200 itself, which may have accumulated blood on an outer surface thereof, sliding against and contacting the compressible seal 150 of the hemostasis valve 130 and/or the elastomeric valve 170. Thus, the elongate shaft 200 may be withdrawn from the side port 120 without directly sliding against and directly contacting the compressible seal 150 of the hemostasis valve 130 and/or the elastomeric valve 170. The elongate shaft 200 may be withdrawn proximally while maintaining the tubular sheath 300 stationary across the hemostasis valve 130 and/or the elastomeric valve 170, or the tubular sheath 300 may be withdrawn together with the elongate shaft 200. In some instances, the elongate shaft 200 may be withdrawn proximally relative to the tubular sheath 300 for a first distance, and thereafter, the elongate shaft 200 and the tubular sheath 300 may be further withdrawn proximally together. In other instances, the elongate shaft 200 may be withdrawn proximally together with the tubular sheath 300 for a first distance, and thereafter, the elongate shaft 200 may be further withdrawn proximally relative to the tubular sheath 300.

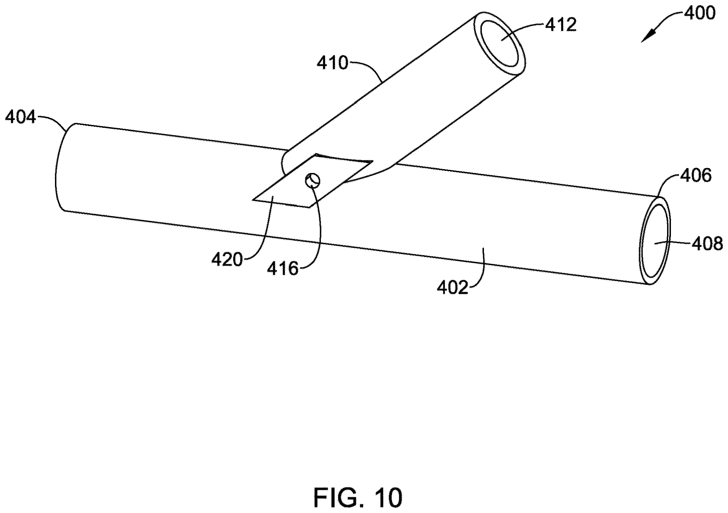

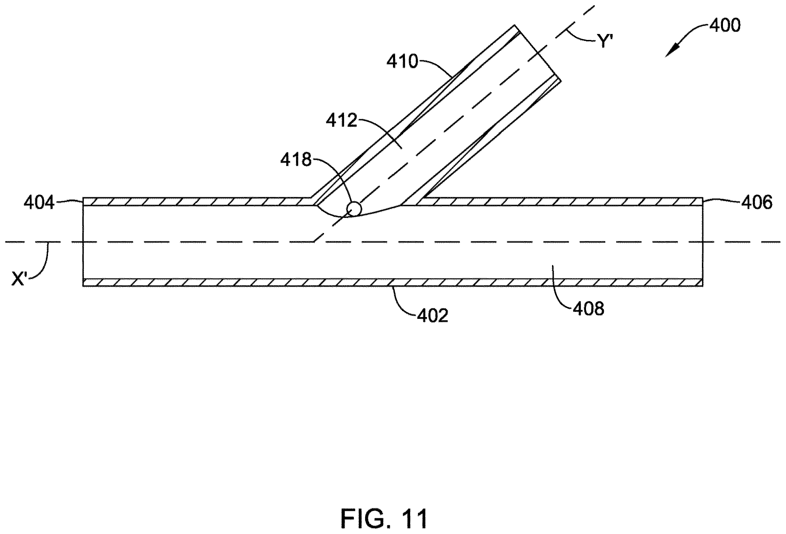

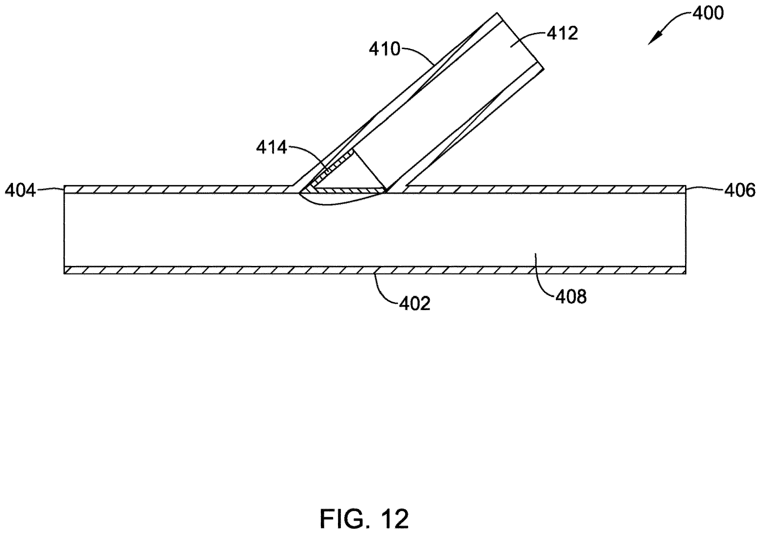

FIGS. 10-12 illustrate aspects of another illustrative adaptor 400 having an alternative elastomeric valve 414 (see FIG. 12). Generally, the elastomeric valve 414 may be similar in form and function to the elastomeric valve 170 described herein. FIG. 11 illustrates a cross-sectional view of the adaptor 400 with the elastomeric valve 414 removed to illustrate some features of the adaptor 400 more particularly. FIG. 12 illustrates a cross-sectional view of the adaptor 400 with the elastomeric valve 414. As shown in FIG. 10, the adaptor 400 may include a body 402 having a first end 404 and a second end 406. The body 402 may define a main passageway 408 extending through the body 402 along a longitudinal axis X'. The main passageway 408 may open out at a first opening at the first end 404 and a second opening at the second end 406. Thus, the main passageway 408 of the adaptor 400 may extend from the first opening at the first end 404 of the body 402 to the second opening at the second end 406 of the body 402.

In some instances, the first end 404 of the body 402 may include a connector (not explicitly shown) and/or the second end 406 of the body may include a connector (not explicitly shown) configured to connect the adaptor 400 to a conduit (e.g., tubing or cannula) of the ECLS system. However, in other instances, the body 402 may be incorporated as a component of a cannula, such as an access cannula. While not explicitly shown, the connectors may be similar in form and function to the connectors 112, 114 shown and described herein. For example, the connectors may be barb fittings configured to be inserted into the lumen of a tubular member (e.g., tubing). The barb fittings may include one or more projecting annular ridges configured to form an interface fit against an inner surface of a tubular member (e.g., tubing). The barb fittings may be any desired size to fit into any desired size of medical tubing. In some instances, the connectors may be the same size and/or configuration as one another. However, in other instances, the connectors may be of differing sizes and/or configurations. In other instances, the connectors may be threaded connections configured to threadingly engage a mating threaded structure, such as a threaded structure provided with a tubular member (e.g., a luer connector, etc.).

The adaptor 400 may also include a side port 410 (i.e., auxiliary port) extending from the body 402. The side port 410 may include a body defining a passageway 412 extending therein. In some instances, the body of the side port 410 may be a monolithic portion of the body 402, or the body of the side port 410 may be formed separately from the body 402 and fixedly secured thereto. The side port 410 may extend from the body 402 at any desired angle. For example, the side port 410, including the passageway 412 thereof, may extend at an acute angle from the body 402 along a longitudinal axis Y', as shown in FIG. 11. However, in other embodiments, the side port 410 may extend at a perpendicular angle from the body 402, if desired. The passageway 412 of the side port 410 may extend along the longitudinal axis Y' and converge with the main passageway 408 of the body 402, such as at an acute angle. The passageway 412 of the side port 410 may converge with the main passageway 408 at a location between the first end 404 and the second end 406 of the body 402.

The side port 410 may include a hemostasis valve (not explicitly shown) configured to permit access to the passageway 412 of the side port 410 and into the main passageway 408. The hemostasis valve may be similar in form and function to the hemostasis valve 130 described herein. An elongate shaft of a medical device may be passed through the hemostasis valve and into the main passageway 408, which defines a portion of a blood conduit, while preventing appreciable amounts of blood from passing out through the hemostasis valve.

The adaptor 400 may also include a vent port (not explicitly shown) having a vent pathway in communication with the passageway 412 of the side port 410. The vent port may be similar in form and function to the vent port 180 described herein. For instance, the vent pathway of the vent port may converge with the passageway 412 of the side port 410 at a location between the hemostasis valve and the main passageway 408. In some instances, the vent pathway of the vent port may converge with the passageway 412 of the side port 410 at a location between the hemostasis valve and the elastomeric valve 414 positioned along the passageway 412 of the side port 410 proximate the main passageway 408.

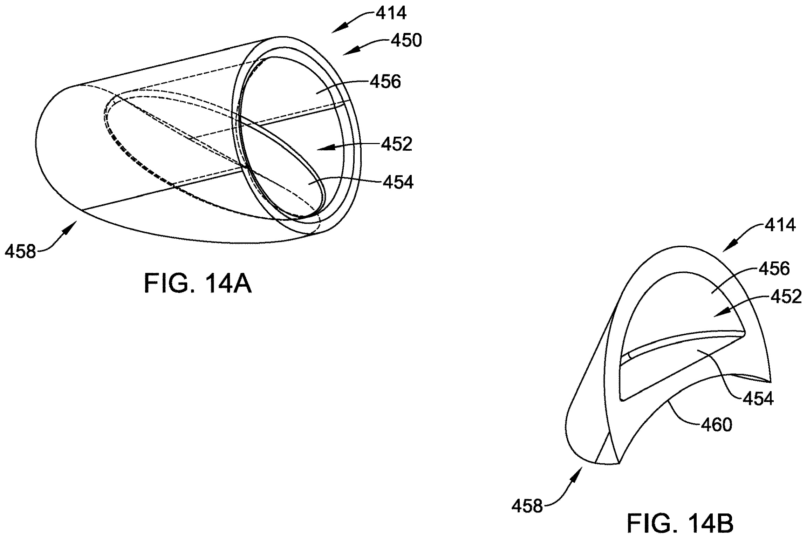

Referring to FIG. 12, the elastomeric valve 414 may be positioned along the passageway 412 of the side port 410 between the hemostasis valve and the main passageway 408. In some instances, the vent port may be positioned between the hemostasis valve and the elastomeric valve 414, with the hemostasis valve spaced apart from the elastomeric valve 414 by a portion of the passageway of the side port 410. The elastomeric valve 414 may be a normally closed valve, having a closed configuration in an equilibrium state such that fluid is substantially prevented from passing through the elastomeric valve 414 in its closed configuration. The elastomeric valve 414 may be configured to allow the elongate shaft of a medical device to pass through the elastomeric valve 414 when the elongate shaft of the medical device is introduced through the passageway 412 of the side port 410 and advanced into the main passageway 408. The elastomeric valve 414 may be sufficiently flexible to deform as the elongate shaft of the medical device is passed therethrough and seal around an outer peripheral of the elongate shaft of the medical device to substantially prevent fluid (e.g., blood) from passing across the elastomeric valve 414 from the main passageway 408. The elastomeric valve 414 may be formed of silicone, polyurethane, thermoplastic elastomer, or other flexible polymeric material. In some embodiments, the elastomeric valve 414 may have a durometer of about 30 - 50 Shore A whereas the body 402 may have a durometer of about 30 - 60 Shore D. In some instances, the elastomeric valve 414 may be a dome valve, a duckbill valve, a slit valve, or any other desired valve configured to seal around an elongate shaft of a medical device when passed therethrough. Some illustrative examples of the elastomeric valve 414 are shown with respect to FIGS. 14-17, which will be described in more detail herein

The elastomeric valve 414 may be positioned proximate the main passageway 408 to reduce the volume of the passageway 412 of the side port 410 between the elastomeric valve 414 and the main passageway 408. Reducing or minimizing the volume of the passageway 412 of the side port 410 between the elastomeric valve 414 and the main passageway 408 may effectively reduce and/or minimize the stagnation or pooling of blood in the passageway 412. For instance, the volume of the passageway 412 of the side port 410 between the elastomeric valve 414 and the main passageway 408, called a stagnation volume, may be 0.03 in³ or less, 0.02 in³ or less, or 0.01 in³ or less, in some embodiments. In some embodiments, a surface of the elastomeric valve 414 facing the main passageway may be flush with the main passageway 408 such that the stagnation volume is substantially zero.

The elastomeric valve 414 may be formed directly within the passageway 412 of the side port 410 and/or the main passageway. For example, the body 402 may include a first aperture 416 (FIG. 10) extending through the sidewall of the body 402 and/or side port 410 from an outer surface to an inner surface thereof and open into the interior of the body 402 and/or side port 410 (e.g., open into the passageway 412) and/or a second aperture 418 (FIG. 11) extending through the sidewall of the body 402 and/or side port 410 from an outer surface to an inner surface thereof and open into the interior of the body 402 and/or side port 410 (e.g., open into the passageway 412). The first and second apertures 416, 418 may be positioned approximately 180° from one another or generally on opposing sides of the body 402. In some embodiments, the first and second apertures 416, 418 may extend through the sidewall of the body 402 and/or side port 410 into the passageway 412 where the side port 410 intersects the body 402. The adaptor 400 may include a first reinforcement member 420 positioned along a first side of the body 402 and/or side port 410 and a second reinforcement member (not explicitly shown) positioned along a second side of the body 402 and/or side port 410. The first reinforcement member 420 may be positioned approximately 180° from the second reinforcement member or generally on opposing sides of the body 402 and the side port 410. The first and second reinforcement members 420 may extend from the body 402 to the side port 410 such that a portion of each of the first and second reinforcement members 420 are secured to the body 402 and the side port 410. In some embodiments, the first and second reinforcement members 420 may be formed as a single monolithic structure with the body 402 and/or side port 410. In other embodiments, the first and second reinforcement members 420 may be formed as a separate component that is fixedly secured to the body 402 and/or the side port 410. The first and second apertures 416, 418 may extend through the reinforcement members 420 such that the apertures 416, 418 extend from an exterior of the adaptor 400 to an interior of the adaptor 400.

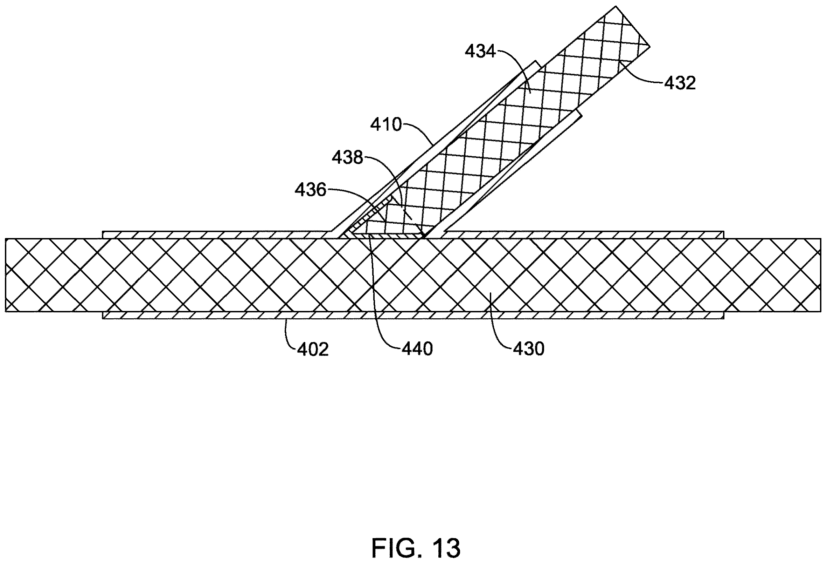

The apertures 416, 418 may be used to inject the material used to form the elastomeric valve 414. The material may be cured within the main passageway 408 and/or the side passageway 412 to form the elastomeric valve 414. Referring additionally to FIG. 13, which illustrates a cross-sectional view of the adaptor 400 during formation of the elastomeric valve 414, one or more mandrels 430, 432 may be positioned within the passageway 408 and/or the side passageway 412 to form a void space within the passageway 408 and/or the side passageway 412 to shape the elastomeric valve 414. The first mandrel 430 may be configured to be inserted into the main passageway 408. The first mandrel 430 may have a length that is greater than a length of the main passageway 408. However, this is not required. In some embodiments, the length of the first mandrel 430 may be less than or approximately equal to the length of the main passageway 408. The first mandrel 430 may be generally cylindrical having an outer diameter that is approximately equal to a diameter of the main passageway 408 (measured at a location spaced from the intersection with the side port 410). Thus, the first mandrel 430 may form a friction fit with the inner surface of the body 402 to prevent the material from the elastomeric valve 414 from migrating or leaking into the main passageway 408.

The second mandrel 432 may be configured to be inserted into the side passageway 412. In some examples, the second mandrel 432 may have a length such that a portion of the second mandrel 432 extends from or beyond the side port 410. However, this is not required. The second mandrel 432 may include a substantially cylindrical first portion 434 and a non-cylindrical second portion 436. The first portion 434 may have an outer diameter that is approximately equal to a diameter of the side passageway 412 (measured at a location spaced from the intersection with the body 402). Thus, the first portion 434 of the second mandrel 432 may form a friction fit with the inner surface of the side port 410 to limit how far the material forming the elastomeric valve 414 extends along the side passageway 410 (e.g., towards the free end thereof).

The second portion 436 may be sized and shaped to form the desired shape of the elastomeric valve 414. Said differently, the size and shape of the elastomeric valve 414 may be defined by the void space between an outer surface of the second portion 436, an inner surface of the side port 410 and the outer surface of the first mandrel 430. In the illustrated embodiment, the second portion 436 may be a diagonally halved cylinder. For example, a first end 438 may have a generally circular cross-sectional shape. Generally, the cross-sectional dimension of the second portion 436 may be less than the diameter of the side port 410 such that there is a gap between an inner surface of the side port 410 and the second portion 436. For example, the diameter of the first end 438 may be less than the diameter of the side passageway 412. The first end 438 may extend generally orthogonal or perpendicular to the longitudinal axis Y' of the side port 410. A second end 440 may define a surface having a generally ellipsoid cross-sectional shape. The surface at the second end 440 may extend at a non-orthogonal or non-perpendicular angle to the longitudinal axis Y' of the side port 410. In some cases, the surface at the second end 440 may extend generally parallel to the longitudinal axis X' of the body 402. However, this is not required. The second end 438 may extend at any angle desired to create an elastomeric valve 414 having the desired shape. In the illustrated embodiment, the surface at the second end 440 is generally planar. This may result in a curved void space between the surface at the second end 440 of the second portion 436 of the second mandrel 432 and the curved outer surface of the first mandrel 430. As the material forming the elastomeric valve 414 is injected into the apertures 416, 418, the material may flow into the empty or void space between an outer surface of the second portion 436, an inner surface of the side port 410 and the outer surface of the first mandrel 430. In some cases, the material may be chemically bond to the inner surface of the side port 410 as the material is cured to secure the elastomeric valve 414 in place.

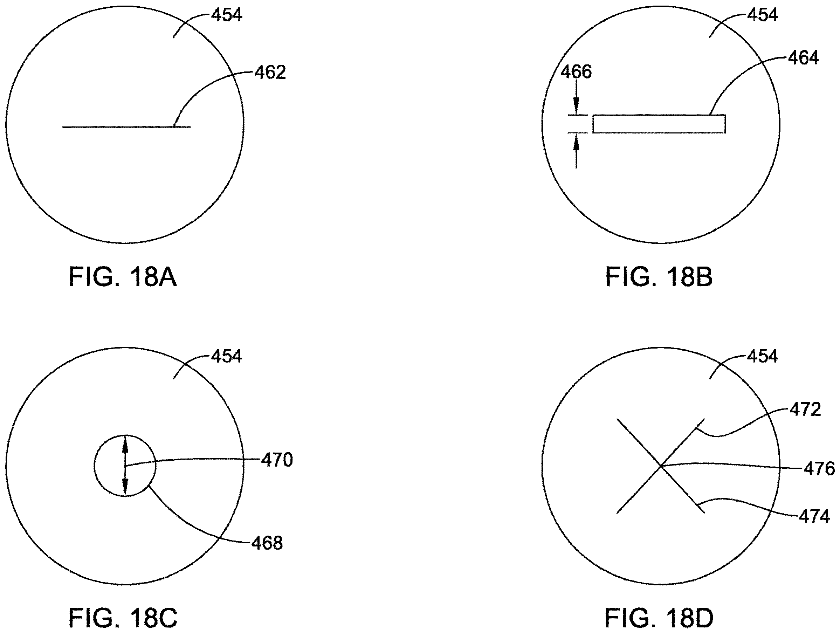

The elastomeric valve 414 may be formed by inserting the first mandrel 430 into and through the main passageway 408. Additionally, the second mandrel 432 may be inserted into the side passageway 412. The second mandrel 432 may be advanced until the surface of the second end 440 is adjacent to but free from contact with the outer surface of the first mandrel 430 (e.g., spaced away from the outer surface of the first mandrel 430 a desired distance to form the valve 414 therebetween). The material to form the elastomeric valve 414 may then be injected in liquid form into the empty or void space between an outer surface of the second portion 436, an inner surface of the side port 410 and the outer surface of the first mandrel 430 through the first and second apertures 416, 418. The material forming the elastomeric valve 414 may be cured in place within the passageway 412 at the convergence of the passageway 412 with the passageway 408 and the mandrels 430, 432 removed. It is contemplated that the elastomeric valve 414 may be chemically bond with the side port 410 during curing of the valve material to maintain the elastomeric valve 414 in the desired position. After the material is cured, one or more slits or openings may be formed in the elastomeric valve 414. Some possible slit or opening configurations are shown in FIGS. 18A-18D. The one or more slits or openings may allow a medical device to pass through the side port 410 and into the body 402 of the adaptor 400.

FIG. 14A is a perspective view of the illustrative elastomeric valve 414 formed with the second mandrel 432 described with respect to FIG. 13. FIG. 14B is a cross-sectional view of the elastomeric valve 414 of FIG. 14A. The elastomeric valve 414 may include a first portion 450 that is formed within the void space between the second portion 436 of the second mandrel 432 and the side port 410 and a second portion 458 that is formed within the void space between the second portion 436 of the second mandrel 432 and the outer surface of the first mandrel 430. The first portion 450 may define a cavity or recess 452 extending therein. The cavity 452 may generally take the same shape as the second portion 436 of the second mandrel 432. For example, the cavity 452 may be bounded by a generally planar surface 454 mirroring the surface at the second end 440 of the second portion 436 of the second mandrel 432 and a curved side wall 456 mirroring the outer surface of the second portion 436 of the second mandrel 432. The second portion 458 of the elastomeric valve 414 may include a generally concave surface 460 facing the main passageway 408 of the body 402. The generally concave surface 460 may have a same radius of curvature as the outer surface of the first mandrel 430 and/or the interior surface of the body 402 defining the main passageway 408. When the elastomeric valve 414 is disposed within the adaptor 400, the generally concave surface 460 may be flush with or in line with the concave side wall of the body 402 defining the passageway 408. Thus, the concave surface 460, which faces the main passageway 408, may be flush with and have the same curvature as the interior surface of the sidewall of the body 402 defining the main passageway 408. Said differently, the main passageway 408 may have a substantially uniform diameter from the first end 404 to the second end 406 thereof. For example, the adaptor 400 may be free from a stagnation volume between the main passageway 408 and the elastomeric valve 414.

A sealing wall of the elastomeric valve 414 may extend across the elastomeric valve 414 and be defined between the concave surface 460 and the planar surface 454. Thus, the concave surface 460 and the planar surface 454 may be defined on opposite sides of the sealing wall extending across the valve 414. As described herein, a medical device may penetrate through the sealing wall as the medical device is advanced through the side passageway 412 into the main passageway 414. As shown in FIG. 14B, the periphery of the sealing wall (closest to the side wall 456) may be thicker than the central portion of the sealing wall due at least in part to the concave shape of the concave surface 460. Although not shown in FIGS. 14A and 14B, one or more slits or openings may be formed in the sealing wall of the elastomeric valve 414. Some possible slit or opening configurations are shown in FIGS. 18A-18D. The one or more slits or openings may allow a medical device to pass through the sealing wall into the main passageway 408 of the adaptor 400.

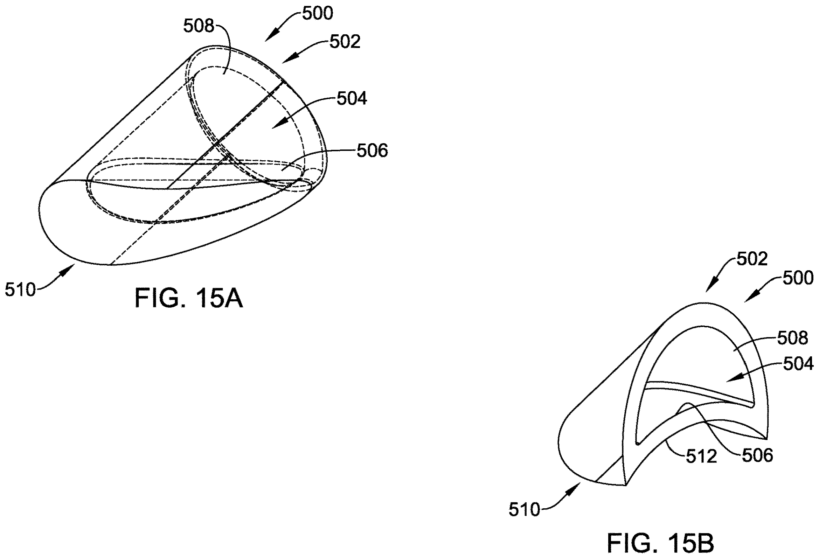

FIG. 15A is a perspective view of another illustrative elastomeric valve 500 formed with a second mandrel having an alternative second portion. FIG. 15B is a cross-sectional view of the elastomeric valve of FIG. 15A. The elastomeric valve 500 may include a first portion 502 that is formed within the void space between the second portion of the second mandrel and the side port 410 and a second portion 510 that is formed within the void space between the second portion of the second mandrel and the outer surface of the first mandrel 430. The first portion 502 may define a cavity or recess 504. The cavity 504 may generally take the same shape as the second portion of the second mandrel. In the illustrated embodiment, the second portion of the second mandrel may be a diagonally halved cylinder. However, the surface at the second end (e.g., corresponding to second end 440) may be generally concave or recessed. Thus, the cavity 504 may be bounded by a generally convex surface 506 mirroring a concave second end surface of the second portion of the second mandrel and a curved side wall 508 mirroring the outer surface of the second portion of the second mandrel. In some cases, the concave second end surface of the second portion of the second mandrel may have a similar radius of curvature as the convex outer surface of the first mandrel 430. However, this is not required. The second portion 510 of the elastomeric valve 500 may include a generally concave surface 512. The thickness of the second portion 510 may be substantially constant from a first side of the cavity 504 to a second side of the cavity 504. The generally concave surface 512 may have a same radius of curvature as the outer surface of the first mandrel 430 and/or the interior surface of the body 402 defining the main passageway 408. When the elastomeric valve 500 is disposed within the adaptor 400, the generally concave surface 512 may be flush with or in line with the concave side wall of the body 402 defining the passageway 408. Thus, the concave surface 512, which faces the main passageway 408, may be flush with and have the same curvature as the interior surface of the sidewall of the body 402 defining the main passageway 408. Said differently, the main passageway 408 may have a substantially uniform diameter from the first end 404 to the second end 406 thereof. For example, the adaptor 400 may be free from a stagnation volume between the main passageway 408 and the elastomeric valve 500.

A sealing wall of the elastomeric valve 500 may extend across the elastomeric valve 500 and be defined between the concave surface 512 and the convex surface 506. Thus, the concave surface 512 and the convex surface 506 may be defined on opposite sides of the sealing wall extending across the valve 500. As described herein, a medical device may penetrate through the sealing wall as the medical device is advanced through the side passageway 412 into the main passageway 414. As shown in FIG. 15B, the curvature of the convex surface 506 may generally match the curvature of the concave surface 512 such that the thickness of the sealing wall may be generally uniform across the extent of the sealing wall. Although not shown in FIGS. 15A and 15B, one or more slits or openings may be formed in the sealing wall of the elastomeric valve 500. Some possible slit or opening configurations are shown in FIGS. 18A-18D. The one or more slits or openings may allow a medical device to pass through the sealing wall into the main passageway 408 of the adaptor 400.

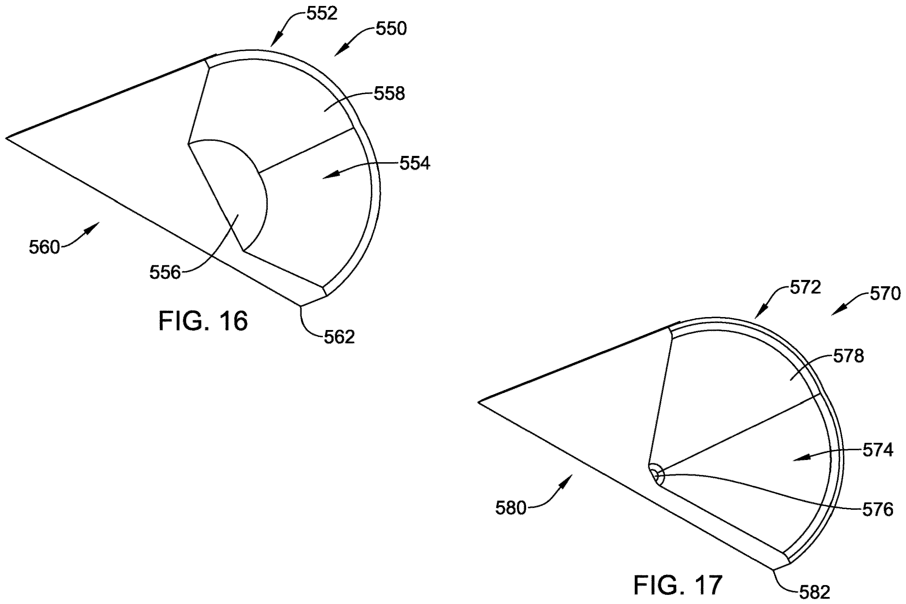

FIG. 16 is a schematic cross-sectional view of another illustrative elastomeric valve 550 formed with a second mandrel having an alternative second portion. The elastomeric valve 550 may include a first portion 552 that is formed within the void space between the second portion of the second mandrel and the side port 410 and a second portion 560 that is formed within the void space between the second portion of the second mandrel and the outer surface of the first mandrel 430. The first portion 552 may define a cavity or recess 554 extending therein. The cavity 554 may generally take the same shape as the second portion of the second mandrel. In the illustrated embodiment, the second portion of the second mandrel may be a truncated cone, having a tapered side surface tapering down to a flat tip having a planar surface extending perpendicular to the longitudinal axis Y'. Thus, the cavity 554 may be bounded by a generally planar surface 556 mirroring the planar surface of the truncated cone of the second portion of the second mandrel and a curved side wall 558 mirroring the outer surface of the second portion of the second mandrel and tapering toward the planar surface 556. An inner diameter of the cavity 554 may reduce in diameter from the opening to the planar surface 556, similar to the angled surface of a cone. The second portion 560 of the elastomeric valve 550 may include a generally concave surface 562 facing the main passageway 408. While the concavity of the surface 562 is not explicitly shown, the surface 562 may be similar in form and function to the concave surface 460 shown and described with respect to FIG. 14B. The thickness of the wall extending across the second portion 560 may vary from a first side of the cavity 554 to a second side of the cavity 554. The generally concave surface 562 may have a same radius of curvature as the outer surface of the first mandrel 430 and/or the interior surface of the body 402 defining the main passageway 408. When the elastomeric valve 550 is disposed within the adaptor 400, the generally concave surface 562 may be flush with or in line with the concave side wall of the body 402 defining the passageway 408. Thus, the concave surface 562, which faces the main passageway 408, may be flush with and have the same curvature as the interior surface of the sidewall of the body 402 defining the main passageway 408. Said differently, the main passageway 408 may have a substantially uniform diameter from the first end 404 to the second end 406 thereof. For example, the adaptor 400 may be free from a stagnation volume between the main passageway 408 and the elastomeric valve 550.

A sealing wall of the elastomeric valve 550 may extend across the elastomeric valve 550 and be defined between the concave surface 562 and the planar surface 556. Thus, the concave surface 562 and the planar surface 556 may be defined on opposite sides of the sealing wall extending across the valve 550. As described herein, a medical device may penetrate through the sealing wall as the medical device is advanced through the side passageway 412 into the main passageway 414. Although not shown in FIG. 16, one or more slits or openings may be formed in the sealing wall of the elastomeric valve 550. Some possible slit or opening configurations are shown in FIGS. 18A-18D. The one or more slits or openings may allow a medical device to pass through the sealing wall into the main passageway 408 of the adaptor 400.

FIG. 17 is a schematic cross-sectional view of another illustrative elastomeric valve 570 formed with a second mandrel having an alternative second portion. The elastomeric valve 570 may include a first portion 572 that is formed within the void space between the second portion of the second mandrel and the side port 410 and a second portion 580 that is formed within the void space between the second portion of the second mandrel and the outer surface of the first mandrel 430. The first portion 572 may define a cavity or recess 574 extending therein. The cavity 574 may generally take the same shape as the second portion of the second mandrel. In the illustrated embodiment, the second portion of the second mandrel may be generally conical. In some cases, the tip of the conical second portion of the second mandrel may be rounded or the conical shape may be truncated very close to the tip thereof. Thus, the cavity 574 may be bounded by an apex 576, which in some instances may be a generally planar surface mirroring a planar surface of the cone (if truncated) of the second portion of the second mandrel and a curved side wall 578 mirroring the outer surface of the second portion of the second mandrel and tapering toward the apex 576. In other embodiments, the end surface at the apex 576 at the tip of the conical portion of the cavity or recess 574 may be hemispherical or a point, for example. An inner diameter of the cavity 574 may reduce in diameter from the opening to the apex 576 of the cavity or recess 574, similar to the angled surface of a cone. The second portion 580 of the elastomeric valve 570 may include a generally concave surface 582 facing the main passageway 408. While the concavity of the surface 582 is not explicitly shown, the surface 582 may be similar in form and function to the concave surface 460 shown and described with respect to FIG. 14B. The thickness of the wall extending across the second portion 580 may vary from a first side of the cavity 574 to a second side of the cavity 574. The generally concave surface 582 may have a same radius of curvature as the outer surface of the first mandrel 430 and/or the interior surface of the body 402 defining the main passageway 408. When the elastomeric valve 570 is disposed within the adaptor 400, the generally concave surface 582 may be flush with or in line with the concave side wall of the body 402 defining the passageway 408. Thus, the concave surface 582, which faces the main passageway 408, may be flush with and have the same curvature as the interior surface of the sidewall of the body 402 defining the main passageway 408. Said differently, the main passageway 408 may have a substantially uniform diameter from the first end 404 to the second end 406 thereof. For example, the adaptor 400 may be free from a stagnation volume between the main passageway 408 and the elastomeric valve 570.

A sealing wall of the elastomeric valve 570 may extend across the elastomeric valve 570 and be defined between the concave surface 582 and the apex 576 and/or conical surface 578. Thus, the concave surface 582 and the apex 576 and/or conical surface 578 may be defined on opposite sides of the sealing wall extending across the valve 570. As described herein, a medical device may penetrate through the sealing wall as the medical device is advanced through the side passageway 412 into the main passageway 414. Although not shown in FIG. 17, one or more slits or openings may be formed in the sealing wall of the elastomeric valve 570. Some possible slit or opening configurations are shown in FIGS. 18A-18D. The one or more slits or openings may allow a medical device to pass through the sealing wall into the main passageway 408 of the adaptor 400.

FIGS. 18A-18D illustrate some example slits and/or openings that may extend through the elastomeric valve 414 to permit passage of an elongate shaft of a medical device therethrough while sealing around the elongate shaft of the medical device. While the slits and/or openings are described with respect to a particular elastomeric valve 414, the slits and/or openings may be used with any of the elastomeric valve structures described herein. FIG. 18A is a schematic view of the planar surface 454 of the elastomeric valve 414. In some embodiments, the elastomeric valve 414 may include a slit 462 which extends from the planar surface 454 through to the generally concave surface 460 (not explicitly shown in FIG. 18A). The slit 462 may be an opening where opposing sides of the slit 462 generally contact one another in absence of a biasing force. The opposing sides of the slit 462 may be moved away from one another to allow a medical device to pass therethrough. For example, a medical device may be advanced through the side passageway 412, through the slit 462 of the elastomeric valve 414, and into the main passageway 408. The slit 462 may seal around an elongate shaft of a medical device when passed therethrough.

FIG. 18B is a schematic view of the planar surface 454 of the elastomeric valve 414. In some embodiments, the elastomeric valve 414 may include a slot 464 which extends from the planar surface 454 through to the generally concave surface 460 (not explicitly shown in FIG. 18B). The slot 464 may be an opening where opposing sides of the slot 464 are spaced a distance 466 from one another such that an opening extends through the elastomeric valve 414 in the absence of a medical device extending therethrough. The opposing sides of the slot 464 may be moved away from one another to allow a medical device to pass therethrough. For example, a medical device may be advanced through the side passageway 412, through the slot 464 of the elastomeric valve 414, and into the main passageway 408. The slot 464 may seal around an elongate shaft of a medical device when passed therethrough. While the slot 464 is illustrated as generally rectangular, the slot 464 may take other cross-sectional shapes, such as, but not limited to, square, circular, oblong, polygonal, triangular, irregular, or the like.

FIG. 18C is a schematic view of the planar surface 454 of the elastomeric valve 414. In some embodiments, the elastomeric valve 414 may include an aperture 468 which extends from the planar surface 454 through to the generally concave surface 460 (not explicitly shown in FIG. 18C). The aperture 468 may have a diameter 470 less than the diameter or cross-sectional dimension of the planar surface 454. However, this is not required. The diameter 470 of the aperture 468 may be enlarged to allow a medical device to pass therethrough. For example, a medical device may be advanced through the side passageway 412, through the aperture 468 of the elastomeric valve 414, and into the main passageway 408. The aperture 468 may seal around an elongate shaft of a medical device when passed therethrough. While the aperture 468 is illustrated as generally circular, the slot 464 may take other cross-sectional shapes, such as, but not limited to, square, rectangular, oblong, polygonal, triangular, irregular, or the like.

FIG. 18D is a schematic view of the planar surface 454 of the elastomeric valve 414. In some embodiments, the elastomeric valve 414 may include a first slit 472 and a second slit 474 which extend from the planar surface 454 through to the generally concave surface 460 (not explicitly shown in FIG. 18D). The first and second slits 472, 474 may intersect at an intersection point 476. It is contemplated that the first and second slits 472, 474 may extend at generally perpendicular or non-perpendicular angles to one another, as desired. The slits 472, 474 may be each be an opening where opposing sides of the respective slit 472, 474 generally contact one another in absence of a biasing force. The opposing sides of the slits 472, 474 may be moved away from one another to allow a medical device to pass therethrough. For example, a medical device may be advanced through the side passageway 412, through the slits 472, 474 of the elastomeric valve 414, and into the main passageway 408. The slits 472, 474 may seal around an elongate shaft of a medical device when passed therethrough. The elastomeric valve 414 may include more than two slits, if so desired.

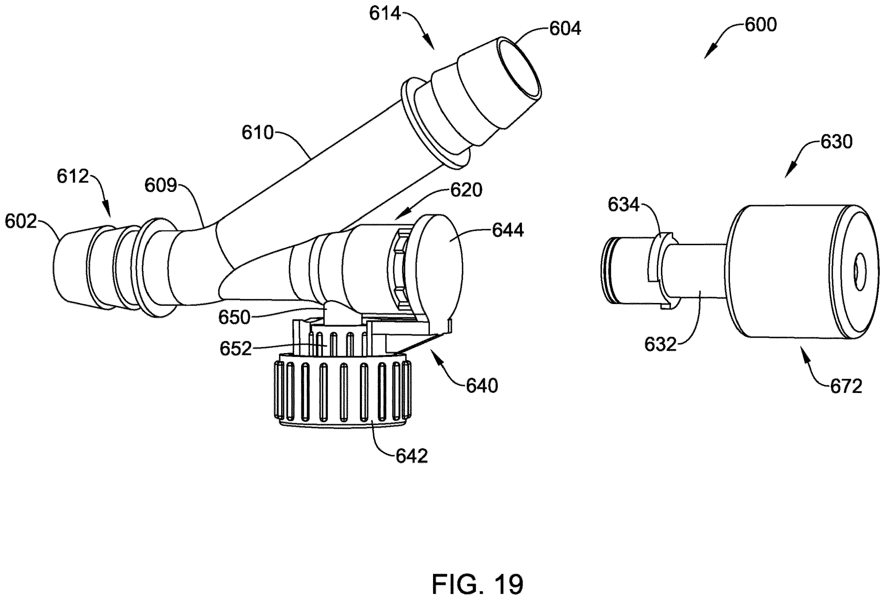

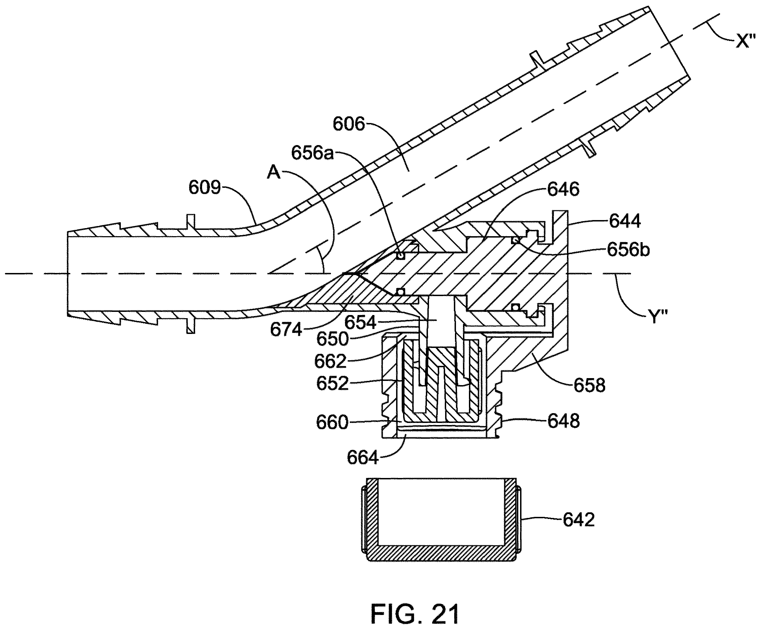

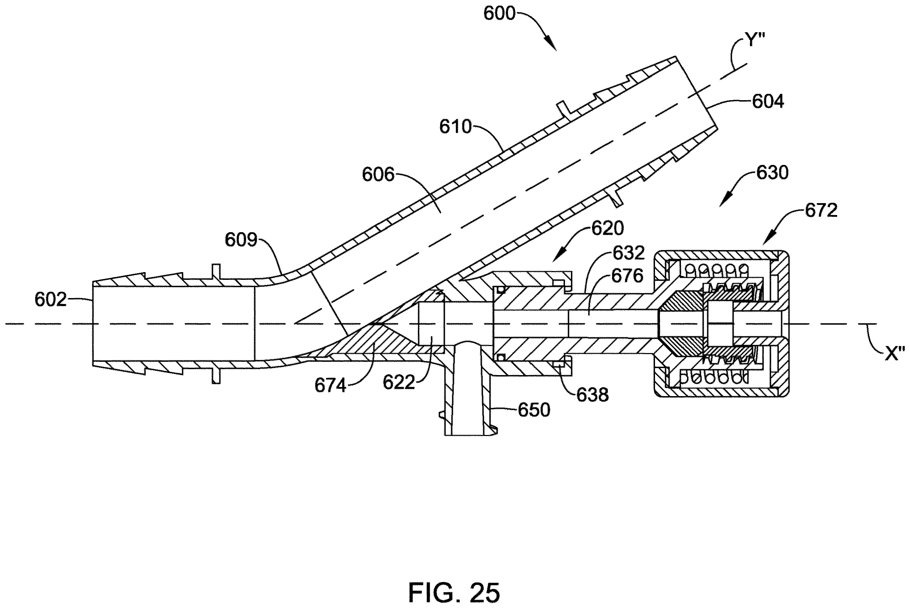

FIGS. 19-26 illustrate aspects of another illustrative adaptor 600. Generally, the adapter 600 may include a removable cap and plug assembly 640 configured to maintain sterility of the adaptor 600. The adaptor 600 may be configured to be releasably coupled to a hemostasis valve 630 for receiving another medical device therethrough. As shown in FIG. 19, the adaptor 600 may include a body 610 having a first end 602 and a second end 604. The body 610 may define a non-linear main passageway 606 (see, for example, FIGS. 21 and 25) extending through the body 610 having a first portion extending along a first longitudinal axis X" and a second portion extending along a second longitudinal axis Y", where the first longitudinal axis X" intersects the second longitudinal axis Y". For example, the body 610 may include a bend or a curved region 609 such that the first longitudinal axis X" and the second longitudinal axis Y" extend at a non-parallel angle A to one another, such as an oblique angle. The main passageway 606 may open out at a first opening at the first end 602 and a second opening at the second end 604. Thus, the main passageway 606 of the adaptor 600 may extend from the first opening at the first end 602 of the body 610 to the second opening at the second end 604 of the body 610. In some instances, the first end 602 of the body 610 may include a connector 612 and/or the second end 604 of the body may include a connector 614 configured to connect the adaptor 600 to a conduit (e.g., tubing or cannula) of the ECLS system. However, in other instances the body 610 may be incorporated as a component of a cannula, such as an access cannula. The connectors 612, 614 are shown as barb fittings configured to be inserted into the lumen of a tubular member (e.g., tubing). The barb fittings may include one or more projecting annular ridges configured to form an interface fit against an inner surface of a tubular member (e.g., tubing). The barb fittings may be any desired size to fit into any desired size of medical tubing. For instance, the barb fittings may be 3/8-inch diameter barb fittings configured to be inserted into a 3/8-inch diameter medical tubing. In other instances, the barb fittings may be of a different size, such as 1/4-inch diameter, 5/16-inch diameter, 1/2-inch diameter, or other desired size to accommodate a corresponding size of medical tubing. In some instances, the connector 612 may be the same size and/or configuration as the connector 614. However, in other instances the connector 612 may be a different size than the connector 614. For example, in some instances, the connector 612 may be a 3/8-inch barb fitting and the connector 614 may be a 1/4-inch barb fitting. In other instances, the connectors 612, 614 may be threaded connections configured to threadingly engage a mating threaded structure, such as a threaded structure provided with a tubular member (e.g., a luer connector, etc.).

The adaptor 600 may also include an auxiliary port 620 extending from the body 610. The auxiliary port 620 may include a body defining a passageway 622 extending therein. In some instances, the body of the auxiliary port 620 may be a monolithic portion of the body 610, or the body of the auxiliary port 620 may be formed separately from the body 610 and fixedly secured thereto. The auxiliary port 620 may extend from the body 610 at any desired angle. For example, the auxiliary port 620, including the passageway 622 thereof, may extend co-linear with the second longitudinal axis Y" of the body 610, as shown in FIG. 21. In other words, the central longitudinal axis of the auxiliary port 620 may be co-axial with the longitudinal axis Y" of the second portion of the main passageway 606. However, in other embodiments the auxiliary port 620 may extend at other angles and/or orientations relative to the body 610, if desired. The passageway 622 of the auxiliary port 620 may extend along the second longitudinal axis Y" and converge with the first longitudinal axis X" of the main passageway 606 of the body 610, such as at an acute angle A. The passageway 622 of the auxiliary port 620 may converge with the main passageway 606 at a location between the first end 602 and the second end 604 of the body 610.

The auxiliary port 620 may be configured to be coupled to a hemostasis valve 630 configured to permit access to the passageway 622 of the auxiliary port 620 and into the main passageway 606. Namely, an elongate shaft of a medical device may be passed through the hemostasis valve 630 and into the main passageway 606, which defines a portion of a blood conduit, while preventing appreciable amounts of blood from passing out through the hemostasis valve 630. In some instance, an elongate shaft of a medical device may be passed through the hemostasis valve 630 and into the second, distal portion of the main passageway 606 parallel to the longitudinal axis Y" without bending the elongate shaft of the medical device.

The adaptor 600 may also include a vent port 650 having a vent pathway 654 in communication with the passageway 622 of the side port 620. For instance, the vent pathway 654 of the vent port 650 may converge with the passageway 622 of the side port 620 at a location between the hemostasis valve 630 and the main passageway 606. In some instances, the vent pathway 654 of the vent port 650 may converge with the passageway 622 of the side port 620 at a location between the hemostasis valve 630 and an elastomeric valve 674 positioned along the passageway 622 of the side port 620 proximate the main passageway 606.

The vent port 650 may include a vent cap 652, which may be removed to open the vent pathway 654 to the ambient environment to vent fluid (e.g., air) from the passageway 622 of the side port 620. In some instances, the vent cap 652 may include a threaded portion (e.g., female threads) threadingly engaged to a mating threaded portion (e.g., male threads) of the vent port 650. During use, the vent cap 652 may be removed and a syringe may be attached to the threads of the vent port 650. The syringe may then be used to remove any air ingression in the passageway 622.

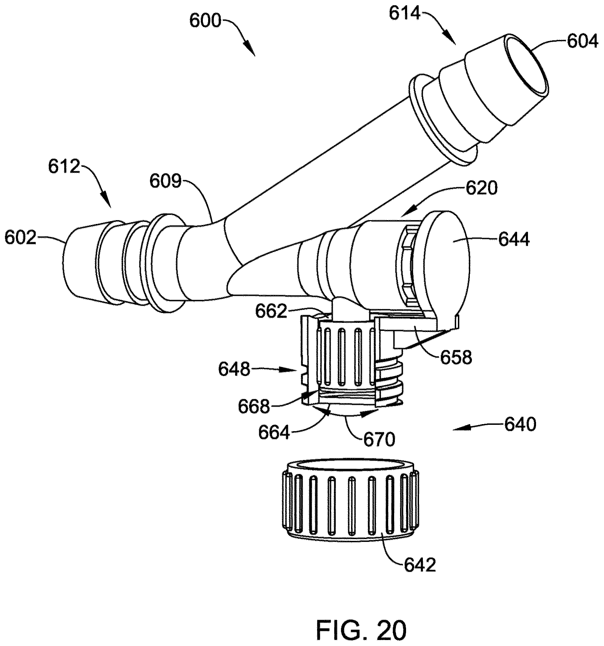

In some embodiments, the auxiliary port 620 may include a cap and plug assembly 640 configured to maintain sterility of the passageway 622 when the hemostasis valve 630 is not connected thereto. Generally, the cap and plug assembly 640 may include a cap 642 configured to releasably couple a plug 644 to the auxiliary port 620. The plug 644 may include a first portion 646 (see, for example, FIG. 21) configured to be at least partially inserted into the passageway 622 and a second portion 648 configured to at least partially surround the vent port 650 and/or vent cap 652. Referring additionally to FIG. 20, the cap 642 may be secured around a perimeter of the second portion 648 of the plug 644 to prevent unintentional rotational and/or axial movement of the plug 644. The cap 642 may be releasably secured to the second portion 648 of the plug 644 using a threaded arrangement. For example, internal threads on the cap 642 may be configured to engage mating external threads on the second portion 648 of the plug 644. However, other coupling mechanisms may be used as desired, such as, but not limited, a bayonet style locking mechanism, friction fits, snap fits, or the like. When it is desired to couple the hemostasis valve 630 with the auxiliary port 620 or the access the vent port 650, the cap 642 may be uncoupled from the second portion 648 of the plug 644 and removed.

Referring additionally to FIG. 21 which illustrates a cross-sectional view of the illustrative adaptor 600 with the cap 642 removed, as described above, the first portion 646 of the plug 644 may be inserted into the passageway 622. The first portion 646 of the plug 644 may be substantially solid and have an outer profile which mirrors an inner diameter of the passageway 622. This may provide a secure fit between the first portion 646 of the plug 644 and the passageway 622. In some embodiments, one or more O-rings 656a, 656b may be positioned about an outer surface of the first portion 646 of the plug 644 to provide a fluid-tight seal between the first portion 646 of the plug 644 and the passageway 622. However, this is not required.

The second portion 648 of the plug 644 may extend generally orthogonal to the first portion 646 and may be coupled to the first portion 646 via an intermediate member 658. The second portion 648 of the plug 644 may define a lumen or opening 660 extending from a first end 662 to a second end 664. The lumen 660 may have a cross-sectional dimension greater than an outer cross-sectional dimension of the vent cap 652 such that the second portion 648 of the plug 644 may be pivotably moved with respect to the vent cap 652. The second portion 648 of the plug 644 may further include an opening 668 extending through a sidewall thereof. The opening 668 may extend from the first end 662 to the second end 664 of the second portion 648. The opening 668 may have an arc length 670 that is larger than the outer cross-sectional dimension of the vent cap 652 such that the second portion 648 of the plug 644 may be pivotably moved with respect to the vent cap 652 while preventing axial movement (e.g., along axis Y") when the second portion 648 is disposed about the vent cap 652.

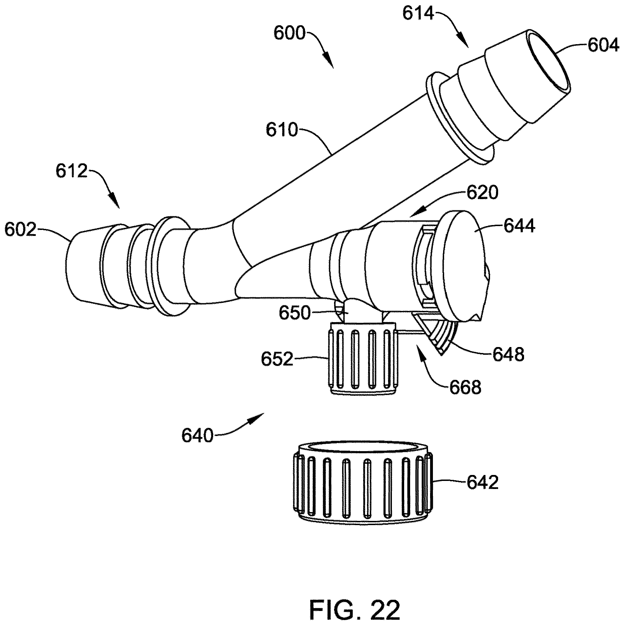

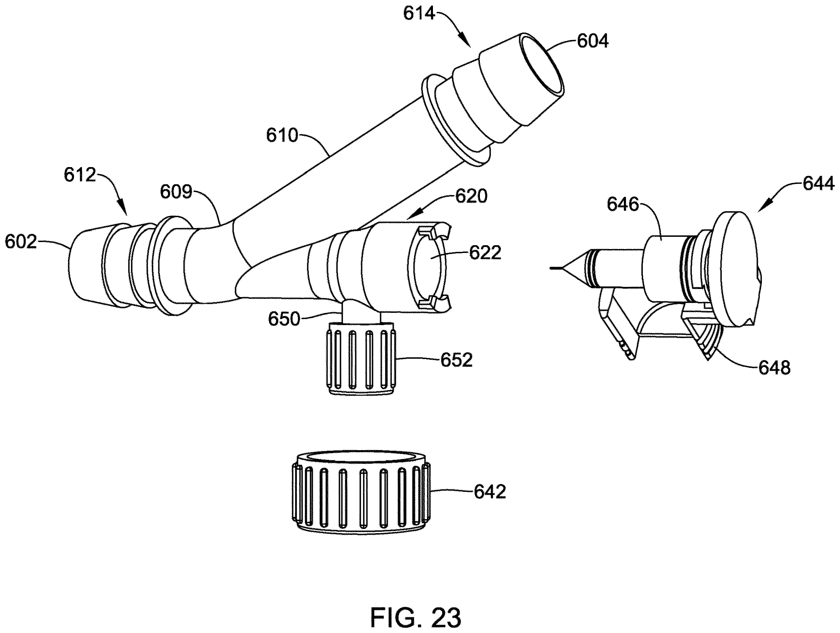

To remove the plug 644 from the passageway 622, after the cap 642 has been uncoupled from the second portion 648 thereof, the plug 644 may be rotated about the second longitudinal axis Y", as shown in FIG. 22. Rotation of the plug 644 may disengage the second portion 648 of the plug 644 from the vent port 650 and/or vent cap 652. For example, the second portion 648 of the plug 644 may include a lateral cut-out allowing the vent port 650, with the vent cap 652 threadably secured thereto, to be passed through the cut-out of the second portion 648 of the plug 644. Once the second portion 648 of the plug 644 has been disengaged from the vent port 650, the plug 644 may be longitudinally displaced along the second longitudinal axis Y" to remove the plug 644 from the passageway 622, as shown in FIG. 23.

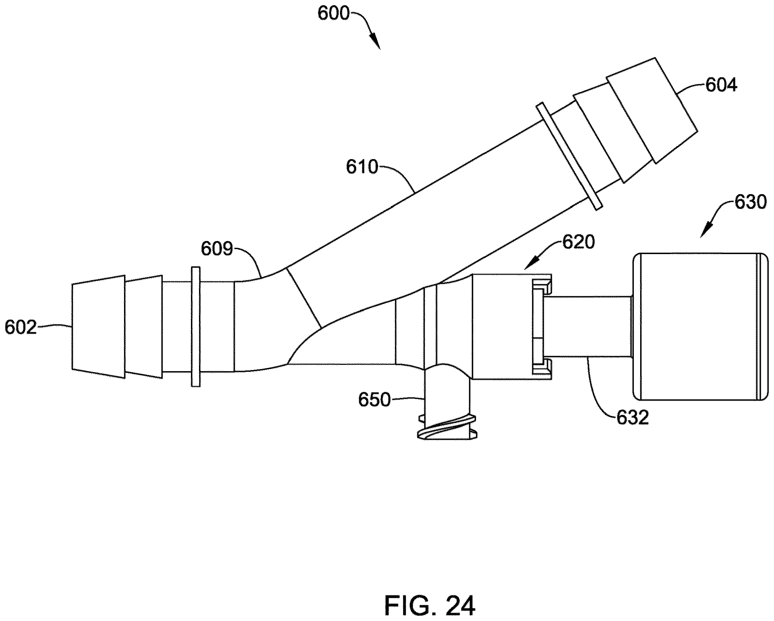

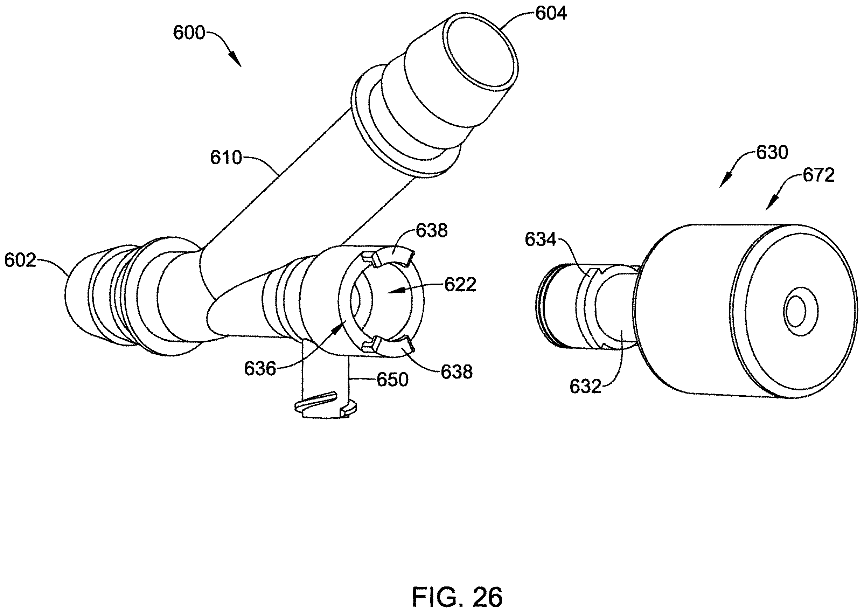

Once the plug 644 has been removed from the passageway 622, the hemostasis valve 630 may be coupled thereto, as shown in FIG. 24. FIG. 25 illustrates a cross-sectional view of the assembled adaptor 600 and hemostasis valve 630. FIG. 26 illustrates a perspective unassembled view of the adaptor 600 and the hemostasis valve 630. The hemostasis valve 630 may include a housing 632 secured to, incorporated with, extending from, or otherwise positioned along the passageway 622 of the auxiliary port 620. For example, the housing 632 of the hemostasis valve 630 may be releasably coupled to the body of the auxiliary port 620. For instance, the housing 632, or at least a portion thereof, may extend into the body of the auxiliary port 620 and be mechanically coupled to an inner surface of the body of the auxiliary port 620. In some examples, the housing 632 may include an O-ring 641 or other sealing member configured to provide a fluid tight seal between an outer surface of the housing 632 and the passageway 622. As shown in FIG. 26, the housing 632 may include one or more, or a plurality of protuberances 634 projecting from an exterior surface of the housing 632 which mate in a corresponding channel, groove, notch or recess formed in the body of the auxiliary port 620 to facilitate securement and/or rotational orientation between the hemostasis valve 630 and the auxiliary port 620. For example, during assembly of the hemostasis valve 630 with the adaptor 600, the one or more protuberances 634 may be aligned one with one or more mating gaps 636 in the auxiliary port 620. The hemostasis valve 630 may be longitudinally advanced until the one or more protuberances 634 abut an end surface of the auxiliary port 620. The hemostasis valve 630 may then be rotated to position the one or more protuberances 634 within a recess defined between an "L" shaped extension 638 and the end surface of the auxiliary port 620. This may releasably secure the hemostasis valve 630 to the auxiliary port 620. When assembled with the auxiliary port 620, a longitudinal axis of the hemostasis valve 630 may extend co-linearly with the first longitudinal axis X" of the body 610 of the adaptor 600.

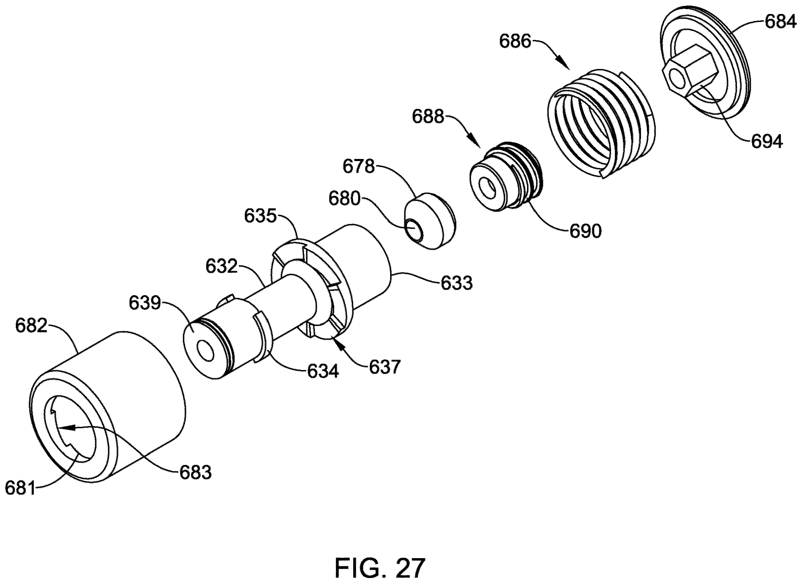

The hemostasis valve 630 may also include a rotatable compression cap assembly 672 that is rotatable relative to the housing 632 and the auxiliary port 620. As will be described further herein in association with FIGS. 27-29, the compression cap assembly 672 may be rotated to compress the seal of the hemostasis valve 630 around an elongate shaft of a medical device inserted through the hemostasis valve 630.

An elastomeric valve 674 may be positioned along the passageway 622 of the auxiliary port 620 between the hemostasis valve 630 and the main passageway 606. In some instances, the vent port 650 may be positioned between the hemostasis valve 630 and the elastomeric valve 674, with the hemostasis valve 630 spaced apart from the elastomeric valve 674 by a portion of the passageway of the auxiliary port 620. The elastomeric valve 674 may be a normally closed valve, having a closed configuration in an equilibrium state such that fluid is substantially prevented from passing through the elastomeric valve 674 in its closed configuration. The elastomeric valve 674 may be configured to allow the elongate shaft of a medical device to pass through the elastomeric valve 674 when the elongate shaft of the medical device is introduced through the passageway 622 of the auxiliary port 620 and advanced into the main passageway 606. The elastomeric valve 674 may be sufficiently flexible to deform as the elongate shaft of the medical device is passed therethrough and seal around an outer peripheral of the elongate shaft of the medical device to substantially prevent fluid (e.g., blood) from passing across the elastomeric valve 674 from the main passageway 606. The elastomeric valve 674 may be formed of silicone, polyurethane, or other flexible polymeric material. In some instances, the elastomeric valve 674 may be a dome valve, a duckbill valve, a slit valve, or any other desired valve configured to seal around an elongate shaft of a medical device when passed therethrough. It is contemplated that the elastomeric valve 674 may vary in size and/or shape and may resemble any of the elastomeric valves described herein.

The elastomeric valve 674 may be positioned proximate the main passageway 606 to reduce the volume of the passageway 622 of the auxiliary port 620 between the elastomeric valve 674 and the main passageway 606. Reducing or minimizing the volume of the passageway 622 of the auxiliary port 620 between the elastomeric valve 674 and the main passageway 606 may effectively reduce and/or minimize the stagnation or pooling of blood in the passageway 622. For instance, elastomeric valve 674 may extend to the main passageway 606 to elimination and/or substantially eliminate stagnation or pooling of blood in the passageway 622. For instance, the volume of the passageway 622 of the auxiliary port 620 between the elastomeric valve 674 and the main passageway 606, called a stagnation volume, may be 0.03 in³ or less, 0.02 in³ or less, or 0.01 in³ or less, in some embodiments. In some embodiments, a surface of the elastomeric valve 674 facing the main passageway 606 may be flush with the main passageway 606 such that the stagnation volume is substantially zero.

Further aspects of the hemostasis valve 630 are illustrated in FIGS. 27-29. The hemostasis valve 630 may include a lumen 676 extending therethrough for the passage of an elongate shaft of a medical device. The lumen 676 of the hemostasis valve 630 may be axially aligned with the longitudinal axis of the passageway 622 of the auxiliary port 620.

The hemostasis valve 630 may include a plurality of components assembled together. For instance, the hemostasis valve 630 may include the compression cap assembly 672 rotatably coupled to the housing 632 of the hemostasis valve 630 to selectively exert a compressive force on a compressible seal 678 positioned in the bore of the housing 632. The compressible seal 678 may include a lumen 680 extending therethrough axially aligned with the lumen 676 of the hemostasis valve 630. The lumen 680 may have a suitable diameter in an open state allowing an elongate shaft of a medical device to pass therethrough. For example, the lumen 680 may have a diameter in an open state of about 15 F (5 millimeters), about 13 F (4.33 millimeters), about 12 F (4 millimeters), about 10 F (3.33 millimeters), or about 9 F (3 millimeters), in some instances. The lumen 680 may be configured to close around an elongate shaft of a medical device via rotation of the compression cap assembly 672 which compresses the compressible seal 678. Thus, the lumen 680 of the compression cap assembly 672 may have a diameter in a closed state which is less than the diameter of the lumen 680 in the open state. In some instances, the compression cap assembly 672 may be configured such that the lumen 680 is able to be completely closed off (e.g., have a diameter of 0 F (0 millimeters) in a completely closed state.

In some instances, the compression cap assembly 672 may be formed of multiple components assembled together. In some instances, forming the compression cap assembly 672 from multiple components may permit the compression cap assembly 672 to be assembled with the housing 632 and the compressible seal 678, but thereafter preclude the compression cap assembly 672 from being removed from the housing 632. For instance, the compression cap assembly 672 may include a knob or wheel 682, a cap 684, a biasing member 686, and a nut 688, formed as separate components, and then assembled when assembled with the housing 632 of the hemostasis valve 630.

Generally, the compressible seal 678 may be inserted into the lumen 676 of the housing 632 from a first end 633 of the housing 632. The lumen 676 of the housing 632 may include a transition from a first diameter to a second diameter. The compressible seal 678 may be configured to abut the transition region between the first diameter and the second diameter. The transition region may be an abrupt stair-step transition or may be a gradual sloped transition, as desired. The nut 688 may be inserted into the lumen 676 of the housing 632 from the first end thereof. The nut 688 may include a threaded region 690 including plurality of external threads configured to threadingly engage a mating threaded portion 692 within the lumen 676 of the housing 632. The biasing member 686 may be disposed about an outer surface of the housing 632 adjacent the first end 633 thereof. Next the cap 684 may be assembled with the knob 682 and nut 688. For example, the cap 684 may include an axially extending post 694 configured to be received with a mating recess 696 of the nut 688. In the illustrated embodiment, the post 694 has a generally hexagonal cross-sectional shape. The mating recess 696 may have a similar cross-sectional shape such that as the cap 684 is rotated, the post 694 drives rotation of the nut 688. It is contemplated that the post 694 and/or the mating recess 696 may take other cross-sectional shapes which allow for rotation of the cap 684 to be translated to rotation of the nut 688, such as, but not limited to, square, rectangular, oblong, polygonal, triangular, or the like. After being assembled together, the knob 682 may be fixedly secured (e.g., adhesive bonded, etc.) to the cap 684 to prevent separation of the components.

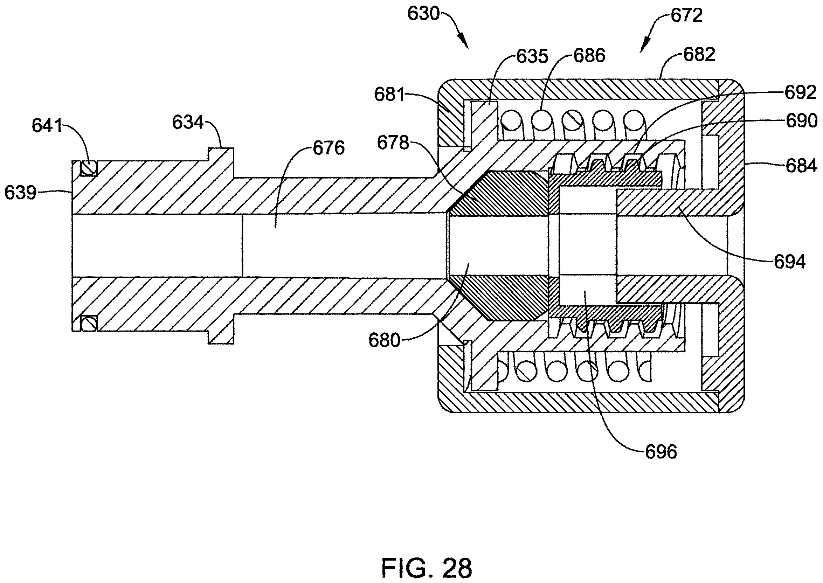

As shown in FIGS. 28 and 29, the housing 632 of the hemostasis valve 630 may include a rim 635, such as a radially outward projecting rim extending around an intermediate region of the housing 632. In some instances, the rim 635 may extend continuously around the first end of the housing 632. In other instances, the rim 635 may extend discontinuously around the first end of the housing 632. The compression cap assembly 672, such as the knob 682 of the compression cap assembly 672, may include a lip 681, such as a radially inward projecting lip 681 extending around the compression cap assembly 672, such as a first end of the knob 682. The lip 681 may be sized such that the rim 635 cannot pass through the opening defined by the lip 681. Such a configuration may prevent the compression cap assembly 672 from being removed from the housing 632 of the hemostasis valve 630.

During use, the compression cap assembly 672 may be rotated relative to the housing 632, thereby threadably advancing the compression cap assembly 672 in the axial direction to exert a compressive force against the compressible seal 678. For instance, rotation of the compression cap assembly 672 may threadingly advance the threaded portion 690 of the nut 688 along the threaded portion 692 of the bore 676 of the housing 632, with an end surface of the nut 688 of the compression cap assembly 672 pressing against an end surface of the compressible seal 678. The compressive force causes compressible seal 678 to deform, thereby reducing the diameter of the lumen 680 through the compressible seal 678. For instance, the compressive force may cause the length of the compressible seal 678 to be reduced as the diameter of the lumen 680 of the compressible seal 678 is reduced.

A surface of the rim 635 may include a plurality of angled notch sections 637. The angled notch sections 637 may be configured to selectively engage a plurality of mating angled notch sections 683 formed in a surface of the lip 681. The mating angled notch sections 637, 683 may be configured to allow the compression cap assembly 672 to be freely rotated in first direction while precluding rotation in a second opposite direction. This may allow the compression cap assembly 672 to be actuated to compress the compressible seal 678 while preventing unintentional release of the compressible seal 678. In some cases, the mating angled notch sections 637, 683 may provide a locking mechanism to provide a temporary hold as the compression cap assembly 672 is rotated. For example, the mating angled notch sections 637, 683 may be configured to provide a temporary hold every 20°, every 30°, every 45°, etc. Other increments may be used, as desired. It is contemplated that as the compression cap assembly 672 is rotated, the angled notch sections 683 of the knob 682 may slide along the angled notch sections 637 of the housing 632. At the end of each angled notch section 637, 683 may be an abrupt stair-step transition which prevents the compression cap assembly 672 from being actuated in the opposing direction. It is contemplated that to reverse the actuation of the nut 688 and release the compressive force of the compressible seal 678, the compression cap assembly 672 may be axially displaced in a direction towards a second end 639 of the housing 632. This may disengage the mating angled notch sections 637, 683 to allow the compression cap assembly 672 to be rotated in the second opposite direction to retract the nut 688. It is contemplated that the axial displacement of the compression cap assembly 672 may be overcome a biasing force of the biasing member 686. In the absence of an axial force on the compression cap assembly 672, the biasing member 686 (such as, but not limited to, a spring) may bias the compression cap assembly 672 in a direction away from the second end 639 of the housing 632 to hold the mating angled notch sections 637, 683 together.

In use, an elongate shaft of a medical device is inserted through the lumen of the compressible seal 678 of the hemostasis valve 630 and into the passageway 622 of the auxiliary port 620. The elongate shaft of the medical device passes through the passageway 622 and is advanced across the elastomeric valve 674 positioned in the passageway 622 of the auxiliary port 620. For example, the elongate shaft may be passed through an opening of the elastomeric valve 674 and cause the elastomeric valve 674 to flex and/or deform to permit the elongate shaft to be advanced therethrough. The elastomeric valve 674 may seal around an outer perimeter of the elongate shaft and substantially prevent blood from entering the passageway 622 of the auxiliary port 620 from the main passageway 606 acting as a portion of the blood pathway. Thereafter, the elongate shaft of the medical device passes into the main passageway 606 of the body 610 of the adaptor 600 and into the blood pathway. The elongate shaft may be advanced along the blood pathway into the body of the patient for a diagnostic and/or therapeutic procedure. The compressible seal 678 may be compressed or tightened around the perimeter of the elongate shaft to prevent blood from leaking past the elongate shaft. For instance, as discussed above, the compression cap assembly 672 may be rotated to exert a compressive force against the compressible seal 678 to seal the compressible seal 678 around the elongate shaft.

The presence of the elastomeric valve 674 proximate the main passageway 606, precludes stagnation of blood along the passageway 622 of the auxiliary port 620, even in the absence of any medical device disposed through the hemostasis valve 630. Thus, the passageway 622 of the auxiliary port 620 may be substantially free of blood throughout use of the adaptor 600.

In some instances, the vent cap 652 may be removed and a syringe may be attached to the vent port 650. The syringe may be used to remove any air ingression in the passageway 622 such that air does not enter the main passageway 606 into the blood pathway.

When it is desired to remove the elongate shaft from the blood pathway, the elongate shaft may be withdrawn from the auxiliary port 620, such as by being retracted from the elastomeric valve 674 and then retracted from the hemostasis valve 630. In some instances, in order to preclude the potential of scraping blood off of the inserted elongate shaft as the elongate shaft is withdrawn from the elastomeric valve 674, a tubular sheath may be advanced distally over the elongate shaft (e.g., surrounding the elongate shaft) through the hemostasis valve 630 and the elastomeric valve 674 to provide an interface between the elongate shaft and the portions of the hemostasis valve 630 and/or the elastomeric valve 674 otherwise contacting the outer surface of the elongate shaft. In some instances, the tubular sheath may be advanced into the main passageway 606 and/or the blood pathway as the tubular sheath is tracked or advanced distally over the elongate shaft. With the tubular sheath extending through the hemostasis valve 630 and/or the elastomeric valve 674, the elongate shaft may be isolated from directly contacting the compressible seal 678 of the hemostasis valve 630 and/or the elastomeric valve 674. Thereafter, the elongate shaft may be withdrawn proximally from the adaptor 600 without the elongate shaft itself, which may have accumulated blood on an outer surface thereof, sliding against and contacting the compressible seal 678 of the hemostasis valve 630 and/or the elastomeric valve 674. Thus, the elongate shaft may be withdrawn from the auxiliary port 620 without directly sliding against and directly contacting the compressible seal 678 of the hemostasis valve 630 and/or the elastomeric valve 674. The elongate shaft may be withdrawn proximally while maintaining the tubular sheath stationary across the hemostasis valve 630 and/or the elastomeric valve 674, or the tubular sheath may be withdrawn together with the elongate shaft. In some instances, the elongate shaft may be withdrawn proximally relative to the tubular sheath for a first distance, and thereafter, the elongate shaft and the tubular sheath may be further withdrawn proximally together. In other instances, the elongate shaft may be withdrawn proximally together with the tubular sheath for a first distance, and thereafter, the elongate shaft may be further withdrawn proximally relative to the tubular sheath.

It is contemplated that prior to advancing a removal sheath through the compressible seal 678 and/or prior to removing the elongate shaft, the radially compressive force of the compressible seal 678 may be removed. To reverse the direction of travel of the nut 688, the compression cap assembly 672 may be actuated towards the second end 639 of the housing 632 to disengage the mating angled notch sections 637, 683. While the compression cap assembly 672 is axially displaced, the compression cap assembly 672 may be rotated in a second direction opposing the first direction to move the nut 688 away from the compressible seal 678 such that the lumen 680 thereof may expand.

It should be understood that this disclosure is, in many respects, only illustrative. Changes may be made in details, particularly in matters of shape, size, and arrangement of steps without exceeding the scope of the disclosure. This may include, to the extent that it is appropriate, the use of any of the features of one example embodiment being used in other embodiments. The scope of the disclosure is, of course, defined in the language in which the appended claims are expressed.

## Claims

1. An adaptor kit for use in a blood conduit of an extracorporeal life support system, the adaptor kit comprising:
an adaptor comprising:
a body defining a non-linear main passageway extending therethrough from a first opening at a first end of the body to a second opening at a second end of the body, the body including a distal end region defining a distal end region of the main passageway and a proximal end region defining a proximal end region of the main passageway, a longitudinal axis of the distal end region of the main passageway converging with a longitudinal axis of the proximal end region of the main passageway;
an auxiliary port extending from the body, a passageway of the auxiliary port converging with the main passageway and having a longitudinal axis extending at an acute angle to the longitudinal axis of the proximal end region of the main passageway; and
an elastomeric valve positioned in the passageway of the auxiliary port adjacent to the main passageway;
a cap and plug assembly configured to be releasably coupled with the auxiliary port of the adaptor; and
a hemostasis valve configured to be releasably coupled to the auxiliary port of the adaptor.

2. The adaptor kit of claim 1, wherein the cap and plug assembly comprises:
a plug having a first portion configured to be received within the passageway of the auxiliary port and a second portion configured to at least partially surround a vent port of the adaptor; and
a cap configured to be releasably coupled to the second portion of the plug.

3. The adaptor kit of claim 2, wherein the plug includes a lateral cut-out configured to allow the vent port to laterally pass therethrough.

4. The adaptor kit of claim 1, wherein the hemostasis valve comprises a housing and a compression cap assembly.

5. The adaptor kit of claim 1, wherein the housing of the hemostasis valve comprises one or more protuberances extending from an exterior of the housing.

6. The adaptor kit of claim 5, wherein the one or more protuberances are configured to be received within a mating slot on the auxiliary port.

7. The adaptor kit of claim 1, wherein the longitudinal axis of the passageway of the auxiliary port is parallel with the longitudinal axis of the distal end region of the main passageway.

8. The adaptor kit of claim 7, wherein the longitudinal axis of the passageway of the auxiliary port is co-axial with the longitudinal axis of the distal end region of the main passageway.

9. The adaptor kit of claim 1, wherein a volume of the passageway of the side port between the elastomeric valve and the main passageway is 0.02 in³ or less.

10. The adaptor kit of claim 1, wherein a surface of the elastomeric valve facing the main passageway is flush with the main passageway of the body.

11. The adaptor kit of claim 10, wherein the surface of the elastomeric valve is a concave surface having a same radius of curvature as an interior surface of the body defining the main passageway.

12. The adaptor of claim 1, wherein the cap and plug assembly is configured to be decoupled from the auxiliary port of the adaptor prior to coupling the hemostasis valve to the auxiliary port of the adaptor.

13. An adaptor for use in a blood conduit of an extracorporeal life support system, the adaptor comprising:
a body defining a main passageway extending therethrough from a first opening at a first end of the body to a second opening at a second end of the body;
a side port extending from the body, a passageway of the side port converging with the main passageway and having a longitudinal axis extending at an acute angle to a longitudinal axis of the body;
wherein the side port includes a hemostasis valve configured to selectively seal around a medical device inserted through the side port into the main passageway; and
an elastomeric valve positioned in the passageway of the side port spaced away from the hemostasis valve, the elastomeric valve located between the hemostasis valve and the main passageway.

14. The adaptor of claim 13, wherein a volume of the passageway of the side port between the elastomeric valve and the main passageway is 0.02 in³ or less.

15. The adaptor of claim 13, further comprising a vent port extending from the side port, wherein the vent port includes a vent passageway converging with the passageway of the side port, wherein the vent port is positioned between the hemostasis valve and the main passageway.

16. The adaptor of claim 13, wherein the hemostasis valve includes a compressible seal having a lumen extending therethrough, and a compression cap configured to exert a compressive force on the compressible seal to reduce a diameter of the lumen through the compressible seal.

17. The adaptor of claim 16, wherein the compression cap includes a stem threadingly engaged to a knob.

18. An adaptor for use in a blood conduit of an extracorporeal life support system, the adaptor comprising:
a body defining a main passageway extending therethrough along a longitudinal axis of the body from a first opening at a first end of the body to a second opening at a second end of the body;
a side port extending from the body, a passageway of the side port converging with the main passageway and having a longitudinal axis extending at an acute angle to the longitudinal axis of the body;
at least one aperture extending through a thickness of a side wall of the side port;
wherein the side port includes a hemostasis valve configured to selectively seal around a medical device inserted through the side port into the main passageway; and
an elastomeric valve positioned in the passageway of the side port spaced away from the hemostasis valve, the elastomeric valve located between the hemostasis valve and the main passageway.

19. The adaptor of claim 18, wherein a surface of the elastomeric valve facing the main passageway is flush with the main passageway of the body.

20. The adaptor of claim 19, wherein the surface of the elastomeric valve is a concave surface having a same radius of curvature as an interior surface of the body defining the main passageway.

21. A method for forming an elastomeric seal in an adaptor for use in a blood conduit of an extracorporeal life support system, the method comprising:
inserting a first mandrel through a main passageway of a body of an adaptor;
inserting a second mandrel into a side passageway of a side port of the adaptor, the side passageway converging with the main passageway;
injecting a material into the side passageway through one or more apertures extending through a side wall of the side port; and
curing the material within the side passageway to form an elastomeric seal.

22. The method of claim 21, wherein a surface of the elastomeric seal facing the main passageway is concave.

23. The method of claim 22, wherein the surface of the elastomeric valve facing the main passageway is flush with an interior wall of the body defining the main passageway.
